# EUROPEAN PATENT APPLICATION

(11) **EP 2 111 864 A2**
(43) Date of publication of application: **28.10.2009**
(21) Application number: 09154213.4
(22) Date of filing: 27.03.2003
(51) Int. Cl.: A61K 31/585, A61P 9/02, A61P 9/04, A61P 11/06, A61P 21/00, A61P 21/04, A61P 25/08, A61P 25/16, A61P 25/28, A61P 27/00

(54) **Therapeutic methods and uses of sapogenins and their derivatives**

(30) Priority: 27.03.2002 AR P020101170; 28.03.2002 US 368178 P; 28.03.2002 WO PCT/GB02/01578
(62) Divisional of application: 03722713.9
(71) Applicant: PHYTOPHARM PLC, Godmanchester, Cambridgeshire PE29 2HY (GB)
(72) Inventor: Rees, Daryl David, Godmanchester Cambridgeshire PE29 2HY (GB); Gunning, Phil, Godmanchester Cambridgeshire PE29 2HY (GB); Orsi, Antonia, Godmanchester Cambridgeshire PE29 2HY (GB); Xia, Zongqin, Shanghai 200025 (CN); Hu, Yaer, Shanghai 200025 (CN)
(74) Representative: Brown, David Leslie

(57) **Abstract**

The invention discloses therapeutic methods and uses of certain steroidal sapogenins, related compounds and derivatives thereof, in the treatment of non-cognitive neurodegeneration, non-cognitive neuromuscular degeneration, motor-sensory neurodegeneration or receptor dysfunction or loss in the absence of cognitive, neural and neuromuscular impairment.

## Description

### Field of the Invention

The present invention relates to therapeutic methods and uses of sapogenins, related compounds and their derivatives.

The uses of the sapogenins, related compounds and their derivatives are in the treatment of non-cognitive neurodegeneration, non-cognitive neuromuscular degeneration, motor-sensory neurodegeneration, or receptor dysfunction or loss. In a further aspect, the invention relates to compositions for use in such treatments.

### Background of the Invention

Cognitive dysfunction is a characteristic of dementia conditions and syndromes, such as Alzheimer's disease (AD), senile dementia of the Alzheimer's type (SDAT), Lewy body dementia and vascular dementia. A lesser degree of cognitive dysfunction is also a characteristic of certain non-dementia conditions and syndromes, such as mild cognitive impairment (MCI), age-associated memory impairment (AAMI), autism and neuroimpairment.

Non-cognitive neurodegeneration (i.e. neurodegeneration in the absence of cognitive dysfunction), non-cognitive neuromuscular degeneration (i.e. neuromuscular degeneration in the absence of cognitive dysfunction) and motor-sensory neurodegeneration are characteristic of conditions and syndromes such as Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS) and multiple sclerosis.

Receptor dysfunction or loss - particularly dysfunction or loss of nicotinic and/or muscarinic acetylcholine receptors and/or dopamine receptors and/or adrenoceptors - is a characteristic of some or all of the above conditions and syndromes. Receptor dysfunction or loss in the absence of cognitive, neural and neuromuscular impairment is also a characteristic of conditions and syndromes such as postural hypotension, chronic fatigue syndrome, asthma, susceptibility to heart failure and macular degeneration.

The above conditions and syndromes are grave and growing problems in all societies where, because of an increase in life expectancy and control of adventitious disease, the demographic profile is increasingly extending towards a more aged population. Agents which can treat, or help in the management or prevention of such disorders, are urgently required.

DE-A-4303214, the disclosure of which is incorporated herein by reference, suggests the use of saponins and sapogenins in the treatment of viral diseases, but with no data that would allow one skilled in the art to select a particular group of compounds for any particular viral disease.

WO-A-99/16786, the disclosure of which is incorporated herein by reference, suggests the use of certain saponins and sapogenins in the treatment of dementia.

WO-A-99/48482, WO-A-99/48507, WO-A-01/23406, WO-A-01/23407, WO-A-01/23408, the disclosures of which are incorporated herein by reference, relate to the use of certain saponins, sapogenins and derivatives thereof in the treatment of cognitive dysfunction and allied conditions.

Chinese Patent Application No. CN-A-1096031, the disclosure of which is incorporated herein by reference, suggests a bioactivity of the spirostane sapogenin, sarsasapogenin, in the two-way regulation of β-adrenergic and M-cholinergic receptors. No specific pharmaceutical activity is suggested. However, in "Synthesis and Applications of Isotopically Labelled Compounds", 1998, pages 315-320, Yi *et al* describe the use of sarsasapogenin in the treatment of senile dementia.

There exist a number of so-called "spectrum" disorders, in which a wide range of combinations of symptoms, in a wide range of relative severities, present themselves. The severity of each symptom and the particular combination of symptoms will vary from individual to individual and according to the stage of progression of the disorder. In the cases of Parkinson's disease, myasthenia gravis, Lambert Eaton disease, postural hypotension and chronic fatigue syndrome, for example, cognitive dysfunction is not a primary symptom, although it may be present as one of a number of possible secondary symptoms. Moreover, these conditions are not viral diseases or dementia. Many of these disorders are so-called "spectrum" disorders, Therefore, in many instances, a treatment for cognitive dysfunction (e.g. dementia) is not necessary.

The present invention is based upon our finding that certain sapogenins and their derivatives, including saponins, have a surprising disease-modifying activity against non-cognitive neurodegeneration, non-cognitive neuromuscular degeneration, motor-sensory neurodegeneration as well as against receptor dysfunction or loss in the absence of cognitive, neural and neuromuscular impairment, and thus actively prevent and reverse the conditions. This finding enables improved treatment of certain non-viral, spectrum and non-spectrum, disorders in which cognitive dysfunction is not a primary symptom.

### Brief Description of the Invention

According to one aspect of the present invention, there is provided the use of the active agents (as herein defined) in the treatment or prevention of, or in the preparation of compositions (e.g. pharmaceutical compositions, foodstuffs, food supplements and beverages) for the treatment or prevention of, (i) non-cognitive neurodegeneration, (ii) non-cognitive neuromuscular degeneration, (iii) motor-sensory neurodegeneration (iv) receptor dysfunction or loss in the absence of cognitive, neural and neuromuscular impairment, in human and non-human animals suffering therefrom or susceptible thereto.

The expression "active agents" refers to compounds of the following general formulae I, II and III, sapogenin derivatives as defined below by reference to derivatives bearing at least one defined X radical substituent, sugar-substituted derivatives of such compounds as defined below, all their stereoisomers and racemic mixtures, all their pharmaceutically acceptable pro-drugs and salts, and all mixtures and combinations thereof: 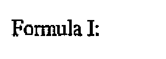 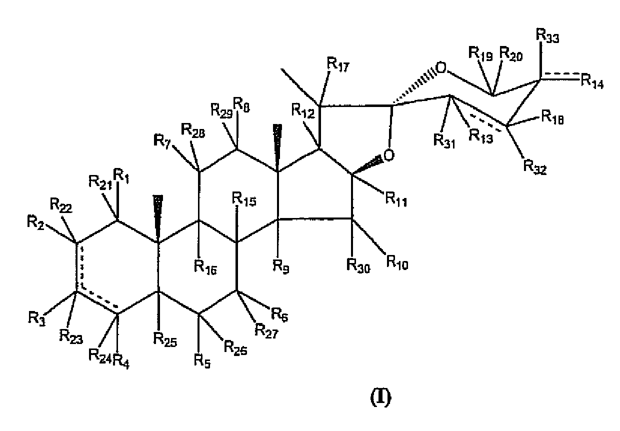 wherein in the general formula (I):
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
   R₃₀, R₃₁, R₃₂, are, independently of each other, either H, OH, =O, halo atom, (Me-S-), (Me-SO-), (Me-SO₂-), N₃-, NH₂-, MeSO₂NH-, alkyl or absent or OR where R = alkyl or acyl
   group;
- R₉, R₁₁, R₁₂, R₁₄, R₁₅, R₁₆, R₁₇, R₂₅, R₃₃ can be either a H, OH, halo atom, (Me-S-), (Me-SO-), (Me-SO₂-), N₃-, NH₂-, MeSO₂NH-, alkyl or absent or OR where R = alkyl or acyl group;
   ..... represents an optional double bond,
   wherein in addition to the above
- either R₃₃ or R₃₄ = alkyl group; 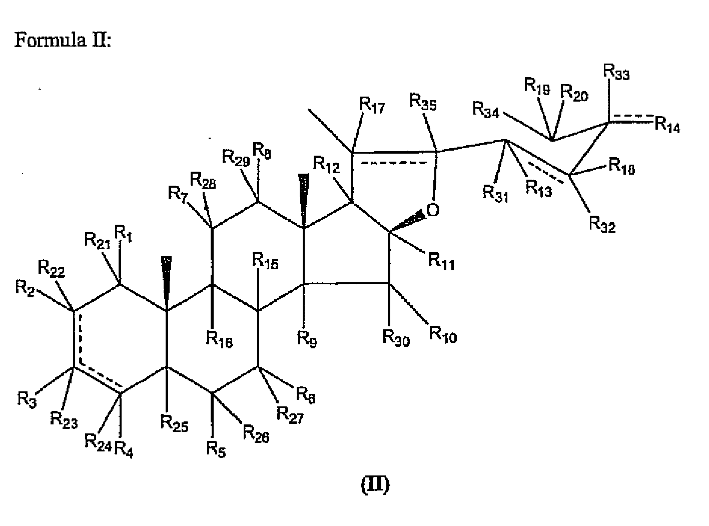
wherein in the general formula (II):
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
   R₃₀, R₃₁, R₃₂, R₃₄, are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
- R₉, R₁₁, R₁₂, R₁₄, R₁₅, R₁₆, R₁₇, R₂₅, R₃₃, R₃₅ can be either a H, OH, halo atom, (Me-S-), (Me-SO-), (Me-SO₂-), N₃-, NH₂-, MeSO₂NH-, alkyl, OR where R = alkyl or acyl group, or absent;
   ..... represents an optional double bond,
   wherein in addition to the above
- either R₃₃ or R₁₄ = alkyl group; 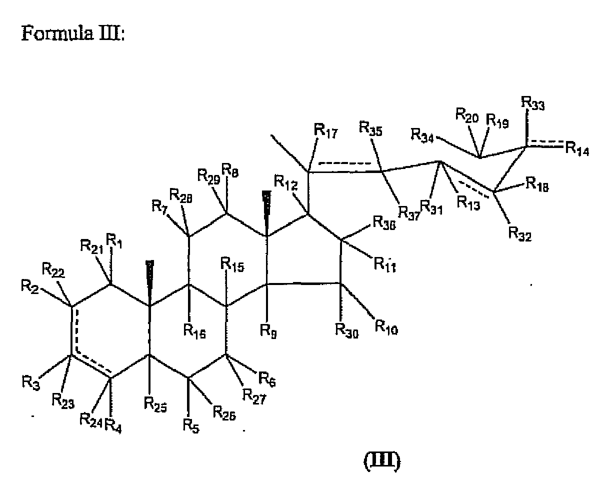
wherein in the general formula (III):
- R₁, R₂, R₃, R₄, R₅, R₆, R₇, R₈, R₁₀, R₁₃, R₁₄, R₁₈, R₁₉, R₂₀, R₂₁, R₂₂, R₂₃, R₂₄, R₂₆, R₂₇, R₂₈,
   R₂₉, R₃₀, R₃₁, R₃₂, R₃₃, R₃₄, R₃₅, R₃₆, R₃₇ are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
- R₉, R₁₁, R₁₂, R₁₅, R₁₆, R₁₇, R₂₅ can be either H, OH, halo atom, (Me-S-), (Me-SO-), (Me-SO₂), N₃-, NH₂-, MeSO₂NH-, alkyl, OR where R = alkyl or acyl group, or absent;
   ..... represents an optional double bond,
   wherein in addition to the above
- either R₃₃ or R₁₄ = alkyl group, and the stereochemistry of R₂₅ is in the β orientation;
sapogenin derivatives, particularly but not exclusively steroidal spirostane sapogenin derivatives, bearing at least one X radical substituent,
wherein X is chosen from the group consisting of :
- halo atom,
- (Me-S-), (Me-SO-), (Me-SO₂-),
- N₃-, NH₂-, MeSO₂NH-, and
- alkyl; and

derivative forms of any of the above compounds, in which the carbon atom at the 3-position (i.e. the carbon to which R₃ is attached, or the corresponding position in the sapogenin derivatives defined above without a formula) or, in the case of Formulae II and III, the 3-position carbon atom, the 26-position (i.e. the carbon to which R₃₄ is attached) or each of the carbon atoms at the 3- and 26-positions, carries an O-sugar moiety wherein the sugar group is a mono-, di- or tri-saccharide.

Sugar-carrying derivative forms of sapogenin active agents are generally referred to in the art as saponins. The expression "carbohydrate" used herein includes particularly such sugar groups.

The active agents used in the present invention are preferably the non-estrogenic steroidal sapogenins, saponins, and derivatives thereof within the terms of the above definition, including all physiologically acceptable pro-drugs and salts thereof.

The active agents may be naturally occurring or non-naturally occurring. Non-naturally occurring active agents may suitably be prepared by modification of side groups and/or side atoms of naturally occurring compounds, as described below and as known in the art.

The invention also provides corresponding methods for the treatment of human and non-human animals, and compositions containing the active agents for use in the said treatment methods.

The active agents of the invention may, if desired, be co-administered with one or more additional active agent, for example one or more agent selected from, but not limited to, cholinesterase inhibitors, dopamine agonists (e.g. L-dopa), COMT inhibitors, MAO-B inhibitors, anti-cholinergics, acetylcholine agonists, serotonin agonists, AMPA receptor agonists, GABA receptor agonists, NMDA receptor agonists, β-adrenoceptor agonists, digoxin, dobutamine, anti-inflarmmatories, neurotrophic factors, statins, adenosine A2a receptor antagonists, aldose reductase inhibitors, immunomodulators, cannabinoid agonists, interferon β or tricyclic anti-depressants.

The active agents may be applied therapeutically or prophylactically to human and non-human animals suffering from, or susceptible to, conditions and diseases that are characterised by non-cognitive neurodegeneration, non-cognitive neuromuscular degeneration, motor-sensory neurodegeneration, or receptor dysfunction or loss. Examples of such conditions and diseases are provided below.

The present invention therefore also provides the use of the active agents (as herein defined) in the treatment or prevention of, or in the preparation of compositions (e.g. pharmaceutical compositions, foodstuffs, food supplements and beverages) for the treatment or prevention of, one or more of said conditions and diseases in human and non-human animals suffering therefrom or susceptible thereto.

### Detailed Description of the Invention

### Examples of Active Agents

The following classes of active agents are particularly mentioned:
1. Compounds of the above general formula I, wherein:
   - R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
      R₃₀, R₃₁, R₃₂ are, independently of each other, either H, OH, =O, halo atom, (Me-S-), (Me-SO-), (Me-SO₂-), N₃-, NH₂-, MeSO₂NH-, alkyl or absent or OR where R = alkyl or acyl group;
   - R₉, R₁₁, R_{12,} R₁₄, R₁₅, R₁₆, R₁₇, R_{25,} R₃₃ can be either a H, OH, halo atom, (Me-S-), (Me-SO-), (Me-SO₂-), N₃-, NH₂-, MeSO₂NH-, alkyl or absent or OR where R = alkyl or acyl group;
      ..... represents an optional double bond,
      wherein in addition to the above
   - either R₃₃ or R₁₄ = alkyl group,
      and the stereochemistry of R₂₅ is in the β orientation;
2. Compounds of the above general formula I, wherein:
   - R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
      R₃₀, R₃₁, R₃₂ are, independently of each other, either H, OH, =O, halo atom, (Me-S-), (Me-SO-), (Me-SO₂), N₃-, NH₂-, MeSO₂NH-, alkyl or absent or OR where R = alkyl or acyl group;
   - R₉, R₁₂, R₁₅, R₁₆, R₁₇ = H,
   - R₁₁, R₁₄, R₂₅, R₃₃ can be either a H, OH, halo atom, (Me-S-), (Me-SO-), (Me-SO₂-), N₃-, NH₂-, MeSO₂NH-, alkyl, or absent or OR where R = alkyl or acyl group;
      ..... represents an optional double bond
      wherein in addition to the above
   - either R₃₃ or R₁₄ = alkyl group,
      and the stereochemistry of R₂₅ is in the β orientation;
3. Compounds of the above general formula I, wherein:
   - R₁= R₂= R₄= R₅= R₆= R₇= R₈= R₁₀ =R₁₁ =R₉= R₁₂= R₁₃= R₁₅ = R₁₆ = R₁₇ = R₁₈ = R₁₉ = R₂₀ =
      R₂₁= R₂₂= R₂₃= R₂₄= R₂₅= R₂₆= R₂₇= R₂₈=R₂₉= R₃₀= R₃₁= R₃₂= R₃₃ = H,
   - either R₃₃ or R₁₄ = CH₃
      ..... represents a single bond,
   - the methyl group at C25 may be either in the R or S configuration
   - the stereochemistry of R₂₅ is in the β orientation and
      wherein in addition to the above
      at least one of R₃ or R₂₃ is a X radical, the possible remaining substituent being H, OH, =O, and OR where R = alkyl or acyl group or absent,
      and X is chosen from the group consisting of:
      - halo atom,
      - (Me-S-), (Me-SO-), (Me-SO₂-), and
      - N₃-, NH₂-, MeSO₂NH-
      - alkyl;
4. Compounds of the above general formula I, wherein:
   - R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
      R₂₁= R₂₂= R₂₃= R₂₄= R₂₅= R₂₆= R₂₇= R₂₈=R₂₉= R₃₀= R₃₁ = R₃₂ = H,
   - R₁₄= R₃₃ = CH₃,
      ..... represents a single bond,
   - the stereochemistry of R₂₅ is in the β orientation and
      wherein in addition to the above
      at least one of R₃ or R₂₃ is a X radical, the possible remaining substituent being H, OH, =O, and OR where R = alkyl or acyl group or absent,
      and X is chosen from the group consisting of:
      - halo atom,
      - (Me-S-), (Me-SO-), (Me-SO₂-), and
      - N₃-, NH₂-, MeSO₂NH-
      - alkyl;
5. Compounds of the above general formula II, wherein
   - R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
      R₃₀, R₃₁, R₃₂, R₃₄ are, independently of each other, either H, OH, =O, halo atom, (Me-S-), (Me-SO-), (Me-SO₂), N₃-, NH₂-, MeSO₂NH-, alkyl, OR where R = alkyl or acyl group, or absent;
   - R₉, R₁₁, R₁₂, R₁₄, R₁₅, R₁₆, R₁₇, R₂₅, R₃₃, R₃₅ can be either a H, OH, halo atom, (Me-S-), (Me-SO-), (Me-SO₂-), N₃-, NH₂-, MeSO₂NH-, alkyl, OR where R = alkyl or acyl group, or absent;
      ..... represents an optional double bond,
      wherein in addition to the above
   - either R₃₃ or R₁₄ = alkyl group, and the stereochemistry of R₂₅ is in the β orientation;
6. Compounds of the above general formula II and carbohydrate derivatives thereof, wherein:
   - R₁, R₂, R₃, R₄, R₅, R₆, R₇, R₈, R₁₀, R₁₃, R_{18,} R₁₉, R_{20,} R₂₁, R₂₂, R₂₃, R₂₄, R₂₆, R₂₇, R₂₈, R_{29,}
      R₃₀, R₃₁, R₃₂ are, independently of each other, either H, OH, =O, halo atom, (Me-S-), (Me-SO-), (Me-SO₂), N₃-, NH₂-, MeSO₂NH-, alkyl, OR where R = alkyl or acyl group, or absent;
   - R₉, R₁₂, R₁₅, R₁₆, R₁₇ = H,
   - R₃₄= either H, OH, =O, and OR where R = alkyl, acyl or carbohydrate and
   - R₁₁, R₁₄, R₂₅, R₃₃, R₃₅ can be either H, OH, halo atom, (Me-S-), (Me-SO-), (Me-SO₂-), N₃-, NH₂-, MeSO₂NH-, alkyl, OR where R = alkyl or acyl group, or absent;
      ..... represents an optional double bond,
      wherein in addition to the above
   - either R₃₃ or R₁₄ = alkyl group,
      and the stereochemistry of R₂₅ is in the β orientation;
7. Compounds of the above general formula II and carbohydrate derivatives thereof, wherein:
   - R₁= R₂= R₄= R₅= R₆= R₇= R₈= R₁₀= R₁₁= R₉= R₁₂= R₁₃= R₁₅ = R₁₆ = R₁₇ = R₁₈ = R₁₉ = R₂₀ =
      R₂₁= R₂₂= R₂₃= R₂₄= R₂₅= R₂₆= R₂₇= R₂₈=R₂₉= R₃₀= R₃₁= R₃₂= R₃₃ = H,
   - R₁₄ = CH₃,
   - R₃₄= -OH or -OR where R= alkyl, acyl or carbohydrate and
      R₃₅=H or is absent
      ..... represents an optional double bond, and
   - the methyl group at C25 may be either in the R or S configuration and and the stereochemistry of R₂₅ is in the β orientation
      wherein in addition to the above
      at least one of R₃ or R₂₃ is a X radical, the possible remaining substituent being H, OH, =O, and OR where R = alkyl or acyl group or absent,
      and X is chosen from the group consisting of:
      - halo atom,
      - (Me-S-), (Me-SO-), (Me-SO₂-), and
      - N₃-, NH₂-, MeSO₂NH-
      - alkyl;
8. Compounds of the above general formula II and carbohydrate derivatives thereof, wherein:
   - R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
      R₂₁= R₂₂= R₂₃= R₂₄= R₂₅= R₂₆= R₂₇= R₂₈= R₂₉= R₃₀= R₃₁= R₃₂= H,
   - R₁₄ = R₃₃ = CH₃,
   - R₃₄= -OH or -OR where R = alkyl, acyl or carbohydrate and
      R₃₅ = H or is absent
      ..... represents an optional double bond, and
      the stereochemistry of R₂₅ is in the β orientation, and
      wherein in addition to the above
      at least one of R₃ or R₂₃ is a X radical, the possible remaining substituent being H, OH, =O, and OR where R = alkyl or acyl group or absent,
      and X is chosen from the group consisting or
      - halo atom,
      - (Me-S-), (Me-SO-), (Me-SO₂-), and
      - N₃-, NH₂-, MeSO₂NH-
      - alkyl;
9. Compounds of the above general formula III, wherein:
   - R₁, R₂, R₃, R₄, R₅, R₆, R₇, R₈, R₁₀, R₁₃, R₁₄, R₁₈, R_{19.} R₂₀, R₂₁, R₂₂, R₂₃, R₂₄, R₂₆, R₂₇, R₂₈,
      R₂₉, R₃₀, R₃₁, R₃₂, R₃₃, R₃₄, R₃₅, R₃₆. R₃₇ are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
   - R₉, R₁₁, R₁₂, R₁₅, R₁₆, R₁₇, R₂₅ can be either H, OH, halo atom, (Me-S-), (Me-SO-), (Me-SO₂), N₃-, NH₂-, MeSO₂NH-, alkyl, OR where R = alkyl or acyl group, or absent;
      ..... represents an optional double bond,
      wherein in addition to the above
   - either R₃₃ or R₁₄ = alkyl group, and
      the stereochemistry of R₂₅ is in the β orientation;
10. Compounds of the above general formula III, wherein:
   - R₁, R₂, R₃, R₄, R₅, R₆, R₇, R₈, R₁₀, R₁₃, R₁₄, R₁₈, R₁₉, R₂₀, R₂₁, R₂₂, R₂₃, R₂₄, R₂₆, R₂₇, R₂₈,
      R₂₉, R₃₀, R₃₁, R₃₂, R₃₃, R₃₅, R₃₆, R₃₇ are, independently of each other, either H, OH, =O, halo atom, (Me-S-), (Me-SO-), (Me-SO₂-), N₃-, NH₂-, MeSO₂NH-, alkyl, OR where R = alkyl, or acyl group, or absent;
   - R₉, R₁₂, R₁₅, R₁₆, R₁₇ = H,
   - R₃₄ = H, OH, =O, halo atom, (Me-S-), (Me-SO-), (Me-SO₂-), N₃-, NH₂-, MeSO₂NH-, alkyl, OR where R = alkyl, acyl or carbohydrate, or absent;
   - R₁₁, R₂₅, can be either H, OH, halo atom, (Me-S-), (Me-SO-), (Me-SO₂-), N₃-, NH₂-, MeSO₂NH-, alkyl, OR where R = alkyl or acyl group, or absent;
      ..... represents an optional double bond,
      wherein in addition to the above
   - either R₃₃ or R₁₄ = alkyl group, and
      the stereochemistry of R₂₅ is in the β orientation;
11. Compounds of the above general formula III and carbohydrate derivatives thereof, wherein:
   - R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
      R₂₁= R₂₂= R₂₃= R₁₄= R₂₅= R₂₆= R₂₇= R₂₈=R₂₉= R₃₀= R₃₁= R₃₂= R₃₃ = H,
   - R₁₄ = CH₃,
   - R₃₄= -OH or -OR where R = alkyl, acyl or carbohydrate and
      R₃₅ = H or is absent
      R₃₇ = H, -OH or =O
      R₃₆= H or -OH
      ..... represents a single bond, and
   - the methyl group at C25 may be either in the R or S configuration and
      the stereochemistry of R₂₅ is in the β orientation,
      wherein in addition to the above
      at least one of R₃ or R₂₃ is a X radical, the possible remaining substituent being H, OH, =O, and OR where R = alkyl or acyl group or absent,
      and X is chosen from the group consisting of :
      - halo atom,
      - (Me-S-), (Me-SO-), (Me-SO₂-), and
      - N₃-, NH₂-, MeSO₂NH-
      - alkyl;
12. Compounds of the above general formula III and carbohydrate derivatives thereof, wherein:
   - R₁= R₂= R₄= R₅= R₆= R₇= R₈= R₁₀=R₁₁= R₉= R₁₂= R₁₃= R₁₅ = R₁₆= R₁₇ = R₁₈ = R₁₉ = R₂₀ =
      R₂₁= R₂₂= R₂₃= R₂₄= R₂₅= R₂₆= R₂₇= R₂₈=R₂₉= R₃₀= R₃₁= R₃₂ = R₁₉ = R₂₀ = H,
   - R₁₄= R₃₃ = CH₃,
   - R₃₄= -OH or -OR where R = alkyl, acyl or carbohydrate and
      R₃₅ = H or is absent
      R₃₇ = H, -OR or =O
      R₃₆ = H or -OH
      ..... represents a single bond, and
   - the methyl group at C25 may be either in the R or S configuration and
      the stereochemistry of R₂₅ is in the β orientation
      wherein in addition to the above
      at least one of R₃ or R₂₃ is a X radical, the possible remaining substituent being H, OH, =O, and OR where, R = alkyl or acyl group or absent,
      and X is chosen from the group consisting of :
      - halo atom,
      - (Me-S-), (Me-SO-), (Me-SO₂), and
      - N₃-, NH₂-, MeSO₂NH-
      - alkyl;
13. Substituted sapogenins, particularly but not exclusively steroidal spirostane sapogenins, wherein at least one OH-group of the sapogenin is substituted with X, chosen from the group consisting of :
   - halo atom,
   - (Me-S-), (Me-SO-), (Me-SO₂-),
   - N₃-, NH₂-, MeSO₂NH-, and
   - alkyl;
14. Sapogenins defined above, particularly but not exclusively steroidal spirostane sapogenins, wherein in the definition of X the halo atom is a fluoro atom;
15. Substituted sapogenins selected from:
   (3β-fluoro-5β, 20α,22α,25R-spirostane), (3,3-dilluoro-5β, 20α,22α,25R-spirostane), (3α-methylsulphonylamino-5β,20α,22α,25R-spirostane), (3α-azido-5β, 20α,22α,25R-spirostane), (3α-amino-5β,20α,22α,25R-spirostane), and their stereoisomers and racemic mixtures, their pharmaceutically acceptable pro-drugs and salts;
16. Substituted sapogenins wherein the parent sapogenin which is then substituted with at least one X radical as defined above is selected from sarsasapogenin, episarsasapogenin, smilagenin, epismilagenin, and anzurogenin-D;
17. Compounds of the general formula Ia: 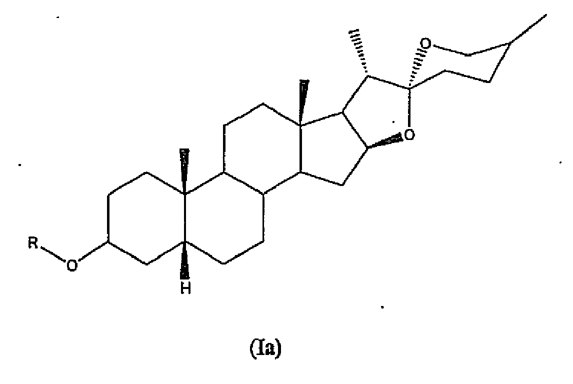 wherein the group R is selected from hydrogen; alkylcarbonyl; alkoxycarbonyl; alkylcarbamoyl; or arylcarbonyl; or sulpho (HO₃S); phosphono ((HO)₂P(O)-); or a mono-, di- or tri-sacaharide; wherein any alkyl group is optionally substituted with aryl, amino, mono- or di-alkyl-amino, a carboxylic acid residue (-COOH), or any combination thereof;
18. Derivative forms of the above compounds as defined as items 1 to 17, in which the 3-position carbon atom or, in the case of Formulae II and III, the 3-position carbon atom, the 26-position carbon atom or each of the carbon atoms at the 3- and 26-positions, carries an O-sugar moiety wherein the sugar group is a mono-, di- or tri-saccharide, for example a mono aldose or ketose having 5 or 6 carbon atoms, preferably in the cyclised furanose or pyranose form, either as the α or β anomer and having D or L optical isomerism, or any di-and tri-oligosaccharide combination thereof; acylated forms of sugar residues are also to be included within the term "sugar"; examples of suitable sugars include glucose, mannose, fructose, galactose, maltose, cellobiose, sucrose, rose, xylose, arabinose, fucose, quinovose, apiose, lactose, galactose-glucose, glucose-arabinose, fucose-glucose, rhamnose-glucose, glucose-glucose-glucose, glucose-rhanrnose, mannose-glucose, glucose-(rhamnose)-glucose, glucose-(rhamnose)-rhamnose, glucose-(glucose)-glucose, galactose-(rhamnose)-galactose and acylated (e.g. acetylated) derivatives thereof.

### In the above compound definitions:

Optional amino, mono-alkyl-amino and di-alkyl-amino substituents of alkyl groups, where present, are preferably a mono-substituent at the α position of the alkyl group.

Optional -COOH substituents of alkyl groups, where present, may be at the terminal or any other position of the alkyl group.

"Alkyl" means an aliphatic hydrocarbon group which may be straight or branched having about 1 to about 20 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. Exemplary alkyl groups include methyl, ethyl, *n*-propyl, *i*-propyl, n-butyl, *i*-butyl, s-butyl, n-pentyl, 3-pentyl.

"Aryl" means any group comprising an aromatic ring or system of fused rings, and preferably contains up to 12 carbon atoms. An exemplary aryl group is the phenyl group. An aryl group may optionally be mono- or poly-substituted, for example by substituents independently selected from halo (e.g. chloro or bromo), alkyl, cycloalkyl, hydroxy, alkoxy, amino, nitro, acylamino, carboxy and alkoxycarbonyl.

"Carboxylic acid residue" means the group -COOH.

"Acyl" means an H-CO- or Alkyl-CO- group wherein the alkyl group is as defined below. Preferred acyls contain a lower alkyl. Exemplary acyl groups include formyl, acetyl, propanoyl, 2-methylpropanoyl, butanoyl and palmitoyl;

"Optionally substituted" means that the said group may be substituted with one or more substituents, which may be the same or different, preferably one or more substituents which individually have a size which is small (e.g. less than about 20% of the largest molecular dimension) in relation to the parent group being substituted; suitable substituents include halo (e.g. chloro or bromo), alkyl, cycloalkyl, hydroxy, alkoxy, amino, acylamino, aryl, aroylamino, carboxy, alkoxycarbonyl, aralkoxycarbonyl, heteroaralkoxycarbonyl, and optionally substituted carbamoyl, preferably subject to the size limitation set out above ;

"Pharmaceutically acceptable" means it is, within the scope of sound medical and veterinary judgement, suitable for use in contact with the cells of humans and lower animals without undue toxicity, irritation, allergic response and the like, and are commensurate with a reasonable benefit/risk ratio. "Pharmaceutically acceptable prodrugs" means those prodrugs of the compounds which are, within the scope of sound medical and veterinary judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds. The term "prodrug" means compounds that are rapidly transformed *in vivo* to yield the parent compound of the above formula for example by hydrolysis in blood. Functional groups which may be rapidly transformed, by metabolic cleavage, in vivo form a class of groups reactive with the carboxyl group. Because of the ease with which the metabolically cleavable groups of the compounds are cleaved in vivo, the compounds bearing such groups act as pro-drugs. A thorough discussion of prodrugs is provided in the following: Design of Prodrugs, H. Bundgaard, ed., Elsevier, 1985; Methods in Enzymology, K. Widder et at, Ed., Academic Press, 42, p.309-396,1985; A Textbook of Drug Design and Development, Krogsgaard-Larsen and H. Bundgaard, ed., Chapter 5; Design and Applications of Prodrugs p.113-19l, 1991; Advanced Drug Delivery Reviews, H. Bundgard, 8, p.1-38, 1992; Journal of Pharmaceutical Sciences, 77, p. 285, 1988; Chem. Pharm. Bull., N. Nakeya et al, 32, p. 692,1984; Pro-drugs as Novel Delivery Systems, T. Higuchi and V. Stella, Vol. 14 of the A.C.S. Symposium Series, and Bioreversible Carriers in Drug Design, Edward B. Roche, ed., American Pharmaceutical Association and Pergamon Press, 1987, which are incorporated herein by reference;

"Pharmaceutically acceptable salts" means the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. See, for example S. M. Berge, et al., Pharmaceutical Salts, J. Pharm. Sci., 66: p.1-19 (1977) which is incorporated herein by reference. Base addition salts can also be prepared by separately reacting the purified compound in its acid form with a suitable organic or inorganic base and isolating the salt thus formed. Base addition salts include pharmaceutically acceptable metal and amine salts. Examples of suitable acid addition salts are those formed with acids selected from hydrochloric, sulphuric, phosphoric and nitric acids. Examples of suitable base addition salts are those formed with bases selected from sodium hydroxide, potassium hydroxide and ammonium hydroxide.

A particularly preferred class of active agents are the compounds of the general formula Ia.

In some of the compounds of formula Ia, the C₂₅ methyl group is in the S configuration; these compounds of the invention are sarsasapogenin and episarsasapogenin or derivatives thereof. In other compounds of formula Ia, the C₂₅ methyl group is in the R configuration; these compounds of the invention are smilagenin and epismilagenin or derivatives thereof.

In the above formula Ia, -OR may, for example, be selected from the following (unless excluded by proviso); hydroxy, cathylate: (ethoxycarbonyloxy), acetate, succinate, cinnamate, ferulate, propionate, butyrate, valerate, isovalerate, caproate, isocaproate, diethylacetate, octanoate, decanoate, laurate, myristate, palmitate, stearate, benzoate, phenylacetate, phenylpropionate, cinnamate, p-nitrobenzoyloxy, 3,5-dinitrobenzoyloxy, p-chlorobenzoyloxy, 2,4-dichlorobenzoyloxy, p-bromobenzoyloxy, m-bromobenzoyloxy, p-methoxybenzoyloxy, phthalyl, glycinate, alaninate, valinate, phenylalaninate, isoleucinate, methioninate, argininate, asparaginate, aspartate, cysteinate, glutamate, histidinate, lysinate, prolinate, serinate, threoninate, tryptophanate, tyrosinate, fumarate or maleate.

Of the compounds of general formula Ia and their pharmaceutically acceptable salts, particularly preferred are the following compounds:
sarsasapogenin
sarsasapogtinin cathylate
sarsasapogenin acetate
sarsasapogenin succinate and pharmaceutically acceptable salts thereof
sarsasapogenin glycinate and pharmaceutically acceptable salts thereof
sarsasapogenin alaninate and pharmaceutically acceptable salts thereof
sarsasapogenin valinate and pharmaceutically acceptable salts thereof
sarsasapogenin phenylalaninate and pharmaceutically acceptable salts thereof
sarsasapogenin isoleucinate and pharmaceutically acceptable salts thereof
sarsasapogenin methioninate and pharmaceutically acceptable salts thereof
episarsasapogenin
episarsasapogenin cathylate
episarsasapogenin acetate
episarsasapogenin succinate and pharmaceutically acceptable salts thereof
episarsasapogenin glycinate and pharmaceutically acceptable salts thereof
episarsasapogenin alaninate and pharmaceutically acceptable salts thereof
episarsasapogenin valinate and pharmaceutically acceptable salts thereof
episarsasapogenin phenylalaninate and pharmaceutically acceptable salts thereof
episarsasapogenin isoleucinate and pharmaceutically acceptable salts thereof
episarsasapogenin methioninate and pharmaceutically acceptable salts thereof
smilagenin
smilagenin cathylate
smilagenin acetate
smilagenin succinate and pharmaceutically acceptable salts thereof
smilagenin glycinate and pharmaceutically acceptable salts thereof
smilagenin alaninate and pharmaceutically acceptable salts thereof
smilagenin valinate and pharmaceutically acceptable salts thereof
smilagenin phenylalaninate and pharmaceutically acceptable salts thereof
smilagenin isoleucinate and pharmaceutically acceptable salts thereof
smilagenin methioninate and pharmaceutically acceptable salts thereof
epismilagenin
epismilagenin cathylate
epismilagenin acetate
epismilagenin succinate and pharmaceutically acceptable salts thereof
epismilagenin glycinate and pharmaceutically acceptable salts thereof
epismilagenin alaninate and pharmaceutically acceptable salts thereof
epismilagenin valinate and pharmaceutically acceptable salts thereof
epismilagenin phenylalaninate and pharmaceutically acceptable salts thereof
epismilagenin isoleucinate and pharmaceutically acceptable salts thereof
epismilagenin methioninate and pharmaceutically acceptable salts thereof.

Of the saponin (R = sugar) compounds of general formula Ia, particularly preferred are the following compounds: sarsasapogenin, episarsasapogenin, smilagenin and epismilagenin in which, in each case, the 3-position carbon atom carries an O-sugar moiety wherein the sugar group is selected from glucose, mannose, fructose, galactose, maltose, cellobiose, sucrose, rhamnose, xylose, arabinose, fucose, quinovose, apiose, lactose, galactose-glucose, glucose-arabinose, fucose-glucose, rhamnose-glucose, glucose-glucose-glucose, glucose-rhamnose, mannose-glucose, glucose-(rhamnose)-glucose, glucose-(rhamnose)-rhamnose, glucose-(glucose)-glucose, galactose-(rhamnose)-galactose and acylated (e.g, acetylated) derivatives thereof.

Further examples of suitable active agents include 16,22-epoxycoprostan-3β-ol, smilagenone, coprosterol and pharmaceutically acceptable pro-drugs and salts thereof.

### Compositions and Uses

The present invention thus enables and provides a method for treating or preventing non-cognitive neurodegeneration, non-cognitive neuromuscular degeneration, motor-sensory neurodegeneration or receptor dysfunction or loss in the absence of cognitive, neural or neuromuscular impairment (particularly but not exclusively in relation to the specific disease states mentioned above) in a human or non-human animal in need thereof, which comprises administering to the said human or non-human animal an effective dosage of an active agent (as defined herein) or a pharmaceutically acceptable salt thereof.

The active agent may be administered in the form of a composition comprising the active agent and any suitable additional component. The composition may, for example, be a pharmaceutical composition (medicament), a foodstuff, food supplement or beverage. Such a composition may contain a mixture of the specified compounds, and/or of their pharmaceutically acceptable salts.

According to a further aspect of the present invention, there is provided a composition having activity against, and for use in treating, non-cognitive neurodegeneration, non-cognitive neuromuscular degeneration, motor-sensory neurodegeneration or receptor dysfunction or loss in the absence of cognitive, neural or neuromuscular impairment in a human or non-human animal, which comprises an effective amount of a compound of the active agent.

The term "pharmaceutical composition" in the context of this invention means a composition comprising an active agent and comprising additionally pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

The terms "foodstuff", "food supplement" and "beverage" used herein have the normal meanings for those terms, and are not restricted to pharmaceutical preparations.

The dosage of the active agent will vary widely, depending on the severity of the symptoms to be treated or prevented. The selection of appropriate dosages is within the ability of one of ordinary skill in this art, without undue burden. The dosage of the active agent may, for example, be greater than about 0.1 mg/kg body weight, for example greater than about 0.3 mg/kg body weight, preferably administered once per day. More typically, the dosage will be between about 1 and about 25 mg/kg, e.g. between about 1 and about 10 mg/kg, preferably administered once per day. For human use, the dosage may conveniently be between about 70 and about 700 mg per day.

"Pharmaceutically acceptable dosage forms" means dosage forms of the compounds or compositions of the invention, and includes, for example, tablets, dragees, powders, elixirs, syrups, liquid preparations, including suspensions, sprays, inhalants, tablets, lozenges, emulsions, solutions, granules, capsules and suppositories, as well as liquid preparations for injections, including liposome preparations. Techniques and formulations generally may be found in Remington, Pharmaceutical Sciences, Mack Publishing Co., Easton, PA, latest edition.

In general, reference herein to the presence of one of a specified group of compounds includes within its scope the presence of a mixture of two or more of such compounds.

This invention provides for the treatment of (i) non-cognitive neurodegeneration, (ii) non-cognitive neuromuscular degeneration, (iii) motor-sensory neurodegeneration, or (iv) receptor dysfunction or loss in the absence of cognitive, neural and neuromuscular impairment, in a human or non-human animal subject suffering from, or susceptible to, any of: Parkinson's disease, postencephalitic Parkinsonism, depression, schizophrenia, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, motor neurone diseases including amyotrophic lateral sclerosis (ALS), multiple sclerosis, postural hypotension, traumatic neurodegeneration e.g. following stroke or following an accident (for example, traumatic head injury or spinal cord injury), Batten's disease, Cockayne syndrome, Down syndrome, corticobasal ganglionic degeneration, multiple system atrophy, cerebral atrophy, olivopontocerebellar atrophy, dentatorubral atrophy, pallidoluysian atrophy, spinobulbar atrophy, optic neuritis, selerosing pan-encephalitis (SSPE), attention deficit disorder, post-viral encephalitis, post-poliomyelitis syndrome, Fahr's syndrome, Joubert syndrome, Guillain-Barre syndrome, lissencephaly, Moyamoya disease, neuronal migration disorders, autistic syndrome, polyglutamine disease, Niemann-Pick disease, progressive multifocal leukoencephalopathy, pseudotumor cerebri, Refsum disease, Zellweger syndrome, supranuclear palsy, Friedreich's ataxia, spinocerebellar ataxia type 2, Rhett syndrome, Shy-Drager syndrome, tuberous sclerosis, Pick's disease, chronic fatigue syndrome, neuropathies including hereditary neuropathy, diabetic neuropathy and mitotic neuropathy, prion-based neurodegeneration, including Creutzfeldt-Jakob disease (CJD), variant CJD, new variant CJD, bovine spongiform encephalopathy (BSE), GSS, FFI, kuru and Alper's syndrome, Joseph's disease, acute disseminated encephalomyelitis, arachnoiditis, vascular lesions of the central nervous system, loss of extremity neuronal function, Charcot-Marie-Tooth disease, susceptibility to heart failure, asthma, and macular degeneration.

The invention therefore includes methods of treating or preventing the above diseases and conditions in a human or non-human animal suffering therefrom or susceptible thereto, which comprises administering to the said human or non-human animal an effective amount of an active agent as defined herein, as well as uses of the active agents in the preparation of compositions for said treatment or prevention.

In the cases of Parkinson's disease, postencephalitic Parkinsonism, postural hypotension, autistic syndrome, chronic fatigue syndrome, myasthenia gravis, and Lambert Eaton disease, as well as any other conditions within the range of disease states to which the present invention relates which are disclosed in, or obvious from, the treatments made available or disclosed in the prior art acknowledged above, the present invention may be subject to the proviso that either symptoms of cognitive dysfunction are absent, or any symptoms of cognitive dysfunction presented by a subject to be treated are secondary or ancillary to the symptoms of non-cognitive neurodegeneration, non-cognitive neuromuscular degeneration, motor-sensory neurodegeneration or receptor dysfunction or loss in the absence of cognitive, neural and neuromuscular impairment.

### Preparation of Compounds for Use in the Invention

Smilagenin, epismilagenin and sarsasapogenin are commercially available materials. Suppliers include, for example, Sigma Aldrich, Research Plus Inc. and Steraloids Inc. Preparative methods for these materials are also to be found in the literature (e.g. a preparation of episarsasapogenin is given in JACS p.5225 (1959)). Episarsasapogenin can be prepared by reduction of sarsasapogenone using a metal hydride reducing agent. Sarsasapogenone can be prepared using the method of Lajis et al, Steroids, 1993, 58, 387-389.

Also, as starting materials, unsubstituted saponins and sapogenins may occur naturally in a range of plant species, notably plants of the genus Smilax, Asparagus, Anemarrhena, Yucca or Agave. Where smilagenin or sarsasapogenin is used in accordance with this invention, it may be in the form of a plant extract, or dry powdered plant material, derived from a plant of the genus Smilax, Asparagus, Anemarrhena, Yucca or Agave.

Methods for preparing the active agents are well known to one of ordinary skill in this art. Examples are shown, for example, in WO-A-021079221 (Examples 5 to 16 therein, which describe the preparation of sarsasapogenin cathylate, episarsasapogenin cathylate, episarsasapogenin succinate, epismilagenin cathylate, episarsasapogenin glycinate hydrochloride, sarsasapogenin glycinate hydrochloride, epismilagenin glycinate hydrochloride, epismilagenin L-alaninate hydrochloride, epismilagenin L-valinate hydrochloride, epismilagenin L-isoleucinate hydrochloride, epismilagenin L-phenylalaninate hydrochloride and epismilagenin L-methioninate hydrochloride). The compounds of formula Ia, other than those with R = H, can be prepared using conventional techniques from compounds in which R = H.

The preferred reaction is a nucleophilic substitution reaction, in which a compound having OH at the 3-position is reacted with a compound of formula
L-R, in which R is selected from alkylcarbonyl; alkoxycarbonyl; alkylcarbamoyl; or arylcarbonyl; or wherein any alkyl group is optionally substituted with aryl, amino, mono-alkyl-amino, di-alkyl-amino, a carboxylic acid residue (-COOH), or any combination thereof; and L is a leaving group, under conditions suitable for nucleophilic substitution.

The compound L-R may, for example, be a carboxylic acid or, if appropriate, an anhydride, or an acyl halide (e.g. an acyl chloride). For example, where R is a cathylate (ethoxycarbonyl) moiety, the compound L-R may suitably be ethyl chloroformate.

The reaction is suitably performed in a base such as pyridine, optionally in the presence of an acid such as hydrochloric acid.

The reaction details for nucleophilic substitution reactions are well known. See, for example, RC Larock, in Comprehensive Organic Transformations, VCH publishers, 1989.

Dihydrosarsasapogenin may be made using the method described in Marker and Rohrmann (1939), Sterols LIII; The structure of the side chain of sarsasapogenin, J. Am. Chem. Soc. 61, pp 846-851. 16,22-Epoxycoprostan-3β-ol may be made using the method described in Scheer et al, (1955), The C-25 isomerism of smilagenin and sarsasapogenin: J. Am. Chem. Soc. 77, pp 641-646.

In the reactions described herein it may be necessary to protect reactive functional groups, for example hydroxy, carboxy or amino groups, where these are desired in the final product, to avoid their unwanted participation in the reactions. Conventional protecting groups may be used in accordance with standard practice. For examples, see TW Green and PGM Wuts, in "Protective Groups in Organic Chemistry", John Wiley & Sons, 1991; JFW McOmie in "Protective Groups in Organic Chemistry", Plenum Press, 1973. For protecting amino substituents in compounds of formula L-R wherein R is amino-substituted, it is preferred to use an alkoxycarbonyl protecting group, whereby the amino function is present as an alkoxycarbonylamino group (preferably t.-butaxycarbonylamino) during the synthetic steps, until deprotection in acid conditions in a dry solvent.

The compound thus prepared may be recovered from the reaction mixture by conventional means. For example, the compound may be recovered by distilling off the solvent from the reaction mixture or, if necessary after distilling off the solvent from the reaction mixture, pouring the residue into water, followed by extraction with a water-miscible solvent and distilling off the solvent from the extract. Additionally, the product can, if desired, be further purified by various well known techniques, such as recrystallisation, reprecipitation, or the various chromatography techniques, notably column chromatography or preparative thin layer chromatography.

### Discussion of the Basis for the Activity

The therapeutic uses underlying the present invention arise from a number of novel observations which are documented in detail in the Examples below. To understand the rationale of the invention, it is useful to summarise the observations and to explain how they predict the therapeutic activities claimed in this invention across the range of active agents defined above.

Smilagenin, epismilagenin, sarsaspogenin and episarsasapogenin restore the loss of muscarinic acetylcholine receptors and adrenoceptors in cells expressing such receptors *in vitro.* These results demonstrate that these compounds restore towards normal cellular receptor loss (Example 1).

Sarsasapogenin, episarsasapogenin cathylate, episarsasapogenin, epismilagenin and smilagenin prevent chemically induced neurodegeneration in rat cortical neurones in *vitro* These results demonstrate that these compounds are neuroprotective and prevent neurodegeneration and neuroimpairment *in vitro* (Example 2).

Sarsasapogenin, smilagenin, 16,22-epoxycoprostan-3□-ol, smilagenone, smilagenin glycinate hydrochloride and coprosterol reverse the chemically induced neurodegeneration in rat cortical neurones *in vitro.* These results demonstrate that the compounds reverse sensory neurodegeneration and neuroimpairment *in vitro* (Example 3).

Smilagenin reverses the chemical induced apoptosis in neurones, .demonstrating that this compound is anti-apoptotic and neuroprotective *in vitro* (Example 4).

Smilagenin and sarsasapogenin increase the neurite outgrowth (neurite number and neurite branching) in rat cortical neurones *in vitro,* demonstrating their neurotrophic effects *in vitro* (Example 5).

Smilagenin and sarsasapogenin prevent and reverse neurotoxin-induced neurodegeneration (neurotoxin 1-methyl-4-phenylpyridinium (MPP⁺) in mesenchephalic dopaminergic neurones *in vitro.* These results demonstrate that these compounds prevent and reverse neurodegeneration and neuroimpairment *in vitro* (Examples 6 and 7).

Sarsasapogenin and smilagenin reverse the chemically induced neurodegeneration in rat spinal motor neurones *in vitro.* These results demonstrate that the compounds reverse neurodegeneration and neuroimpairment of motor neurones *in vitro* (Example 8).

Sarsaspogenin, episarsasapogenin cathylate and smilagenin reduce the number of wrong responses in a cognitive ability test *in vivo* in aged rats, which correlates with an increase in muscarinic acetylcholine receptor density in the brains of aged rats following treatment with the compounds tested. These results demonstrate that the compounds reverse neuroimpairment *in vivo* (Example 9).

Smilagenin and sarsasapogenin reverse the decline of muscarinic acetylcholine and dopamine receptors and the decline in brain derived neurotrophic factor (BDNF) in aged animals. These results demonstrate that the compounds reverse motor-sensory neurodegeneration and neuroimpairment and are neurotrophic *in vivo* (Example 9).

Episarsasapogenin cathylate, sarsasapogenin cathylate, episarsasapogenin and epismilagenin, reduce the number of wrong responses in a cognitive ability test *in vivo* in young rats exposed to neurotoxic agents (ibotenic acid and amyloid □, and increase in muscarinic acetylcholine receptor density in the brains. These results demonstrate that the compounds reverse neuroimpairment *in vivo* (Example 10).

Smilagenin and sarsasapogenin improve survival and motor-sensory neurodegeneration and neuroimpairment in a mouse model of amyotrophic lateral sclerosis (ALS) and Charcot-Marie-Tooth disease (Example 11).

In summary, the compounds have been found to slow or reverse certain aspects of neuronal degeneration. These include the reversal of adverse changes in the cell body, atrophy of neuronal extensions (neurites), reduction in release of neurotrophic factors such as neurotrophins (e.g. BDNF, NGF, NT-3, NT4/5), TGF-β super-family neurotrophic factors (e.g. GDNF) and neurokines (e.g. CNTF, LIF), and neuronal toxicity or death (apoptosis). The compounds are strongly neuroprotective, stimulative of neurite outgrowth, and preventive of neurotoxicity. The compounds have also been found to slow or reverse decreases in cholinergic and dopaminergic function, for example, decreases in muscarinic acetylcholine and dopamine receptor density. Furthermore, we have found that the neuroprotective and the reversal of receptor loss effects are actively regulated effects, in which past deterioration is reversed towards the normal or young state with protection against continued deterioration. Still further, we have found that the reversal of apoptotic effect of the compounds appears to be regulated in the non-neoplastic domain of cell life, and does not appear likely to trigger neoplasia.

The above data, taken together, indicate activity against the disease states already listed in this application. Furthermore, the above data indicates a likely absence of severe or life-threatening side effects such as cancer. The active agents are typically non-oestrogenic.

The prior art acknowledged above shows the sound basis of prediction for extension of the above observations and sound predictions of therapeutic activity to the related chemical structures and derivatives embraced by the term "active agents" in the present invention.

It is well known in the art and in pharmacology that sugar, ester and other groupings at suitable locations on steroid molecules, particularly at the 3- and/or 26-positions, can be easily cleaved off by *in vivo* hydrolysis, and the same effects would be expected to be observed at other carbon atoms of the molecules. Further, it is well known in the art and in pharmacology that salts, free acids and free bases within the terms of the expression "active agents" as used herein are readily convertible *in vivo* between each other according to the pH of the body fluid in which the active agent is present Further, it is well known that side group substituents, in a wide range of forms, can be present in a complex carbon skeleton without substantially adversely affecting the pharmacological activity of the structure, particularly when the side groups are small in comparison with the overall size of the molecule.

For all these reasons, the claims of beneficial pharmacological activity made in the present application are seen to be reasonable and based on sound and credible prediction from the test data assembled and presented herein.

Without wishing to be bound by theory, it is believed that one physiological effect of the active agents is the ability to increase the synthesis, or release of, or to reduce the rate of degradation of, neurotrophic factors such as brain derived neurotrophic factor and/or nerve growth factor or their receptors. These effects on growth factors might be due to an effect of the compound on a cytosolic or nuclear receptor, or the binding of a compound to a promoter region with a consequent effect directly on the rate of production of mRNA for the growth factor, or as a consequence of increasing the production of another material factor.

In addition, the compounds appear to regulate receptors. For example, some of these compounds have been found to prevent or reverse the loss of muscarinic acetylcholine or dopamine receptors in the brain. It is believed that the compounds function by rectifying a deficiency in receptor number or function or turnover.

### Brief Description of the Drawings

In order to illustrate the invention further by way of non-limiting example, reference will now be made to the accompanying drawings and to the Examples which follow.

In the drawings:
Figure 1 shows the effect of epismilagenin acetate on m3 and β2 adrenoceptor density at day 5 in a CHO-β2/m3 co-transfected cell line;
Figure 2 shows the effects of sarsasapogenin, episarsasapogenin cathylate and smilagenin on glutamate induced neurodegeneration in rat primary cortical neurons;
Figure 3 shows the effects of sarsasapogenin, episarsasapogenin cathylate and smilagenin on the learning ability and memory of aged rats;
Figure 4 shows the effects of sarsasapogenin, episarsasapogenin cathylate and smilagenin on muscarinic receptor number;
Figure 5 shows the survival profile of SOD-1 mice following oral administration of smilagenin; and
Figure 6 shows the survival profile of pmn mice following oral administration of sarsasapogenin.

### Detailed Description of the Drawings and Examples

### EXAMPLE 1

### Restoration of receptor loss in vitro

The effects of epismilagenin cathylate, sarsasapogenin cathylate, episarsasapogenin cathylate, episarsasapogenin succinate, epismilagenin acetate and sarsasapogenin on the expression of muscarinic acetylcholine receptor (m) in CHO cells or β2 and m3 receptors in CHO cells transfected with either a vector for the m receptor or co-transfected with the vector for β2 and m3 receptors were investigated.

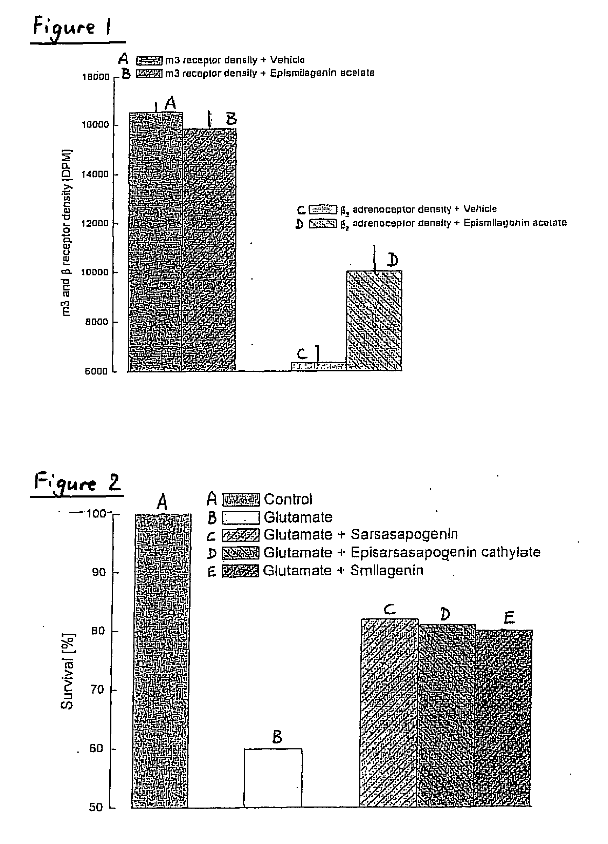

The results are illustrated in Table 1 below and in Figure 1 of the drawings. Over the culturing period of CHO cells transfected with a vector for the m receptor, treatment with epismilagenin cathylate, sarsasagogenin cathylate, episarsasapogenin cathylate, episarsasapogenin succinate and sarsasapogenin each prevents the decrease in m receptor number. Over the culturing period of the CHO cells co-transfected with the vector for β2 and m3 receptors, the density of the m3 receptor did not alter; whereas the density of the β2 adrenoceptors decreased. Incubation with epismilagenin acetate (Figure 1) did not significantly alter the density of m3 receptors; but significantly prevented the decrease in β2 adrenoceptors .

**Table 1 - Effect of epismilagenin cathylate, sarsasapogenin cathylate, episarsasapogenin cathylate, episarsasapogenin succinate and sarsasapogenin on restoration of m acetylcholine receptor density.**

| **Compound** | **Concentration [microM]** | **Activity** |
|---|---|---|
| epismilagenin cathylate | 10 | + + |
| sarsasapogenin cathylate | 10 | + + |
| episarsasapogenin cathylate | 10 | + + |
| episarsasapogenin succinate | 10 | + + |
| Sarsasapogenin | 10 | + + |

Thus, the experiments indicate that each of epismilagenin cathylate, sarsasapogenin cathylate, episarsasapogenin cathylate, episarsasapogenin succinate, epismilagenin acetate and sarsasapogenin were able to prevent decline in receptor number with time and also tend to restore receptor number to normal levels when given to cells in which the receptor level is depressed.

### EXAMPLE 2

### Neuroprotective effect of sarsasapogenin, episarsasapogenin cathylate, episarsasapogenin, epismilagenin and smilagenin in neurones

The objective of this study was to examine the effects of sarsasapogenin, episarsasapogenin cathylate, episarsasapogenin, epismilagenin and smilagenin on the survival of rat primary cortical neurones exposed to glutamate, which is known to induce neurodegeneration.

Rat cortical neurones were cultured for 10 days; at day 10 the medium was changed to a serum-free defined medium. On day 12, 24 hours before glutamate exposure, cultures were washed and medium was replaced with fresh medium containing positive control (β-oestradiol) test compounds (sarsasapogenin, episarsasapogenin cathylate, episarsasapogenin, epismilagenin and smilagenin) or vehicle control (DMSO, 0.25%) or diosgenin as negative control.

On day 13, cultures were exposed to glutamate. After the incubation period, the cultures were washed with and placed in fresh medium, supplemented with relevant compounds or vehicle to evaluate their protective effects, 24 h after glutamate exposure.

Neuronal cell survival was evaluated by measuring lactate dehydrogenase (LDH) activity released in the media 24 h after test compound treatment or glutamate + test compound exposure, using the CytoTox 96 non-radioactive kit and quantitated by measuring wavelength absorbance at 450 nm.

Following exposure of rat primary cortical cultures with glutamate, there was a significant degeneration of cortical neurones, 24 h post-treatment, demonstrated by an increase in lactate dehydrogenase release into the culture medium.

In primary cortical cultures pre-treated with the compounds for 24 h, there was also a significant reduction in the glutamate-induced neurodegeneration (Figure 2; Table 2).

**Table 2 - Effect of sarsasapogenin, episarsasapogenin cathylate, episarsasapogenin, epismilagenin and smilagenin on prevention of glutamate-induced neurodegeneration**

| **Conditions** | **Mean ± s.e.m (%)** |
|---|---|
| Control | 100 |
| + glutamate | 66 ± 3 |
| + glutamate + sarsasapogenin (30 nM) | 79 ± 3 |
| Control . | 100 |
| + glutamate | 65 ± 3 |
| + glutamate + episarsasapogenin cathylate (30 nM) | 74 ± 3 |
| Control | 100 |
| + glutamate | 68 ± 4 |
| + glutamate + episarsasapogenin (30 nM) | 88 ± 3 |
| control | 100 |
| + glutamate | 71 ± 2 |
| + glutamate + epismilagenin (30 nM) | 79 ± 2 |
| Control | 100 |
| + glutamate | 68 ± 4 |
| + glutamate + smilagenin (30 nM) | 91 ± 4 |
| Control | 100 |
| + glutamate | 68 ± 4 |
| + glutamate + diosgenin (30 nM) negative control | 72 ± 4 |

Sarsasapogenin, episarsasapogenin cathylate, episarsasapogenin, epismilagenin and smilagenin all displayed significant neuroprotective effects against glutamate-induced neurodegeneration in rat primary cortical neurones *in vitro.*

### EXAMPLE 3

### Reversal of neurodegeration by sarsasapogenin, smilagenin, 16,22-epoxycoprostan-3β-ol, smilagenone, smilagenin glycinate hydrochloride and coprosterol in neurones

As above mentioned, exposure of rat primary cortical cultures to glutamate (100 µM; 10 min) caused an increase in lactate dehydrogenase (LDH) activity measured after 24 h, indicating a significant neurodegeneration. Treatment with 17β-oestradiol after glutamate exposure produced a significant decrease in the LDH activity compared to neurones exposed to glutamate, suggesting a significant neuroprotective effect. Similarly, treatment with sarsasapogenin, smilagenin, 16,22-epoxycoprostan-3β-ol, smilagenone, smilagenin glycinate hydrochloride and coprosterol produced a significant decrease in the LDH activity compared to neurones exposed to glutamate, suggesting a significant neuroprotective effect (Table 3).

**Table 3 - Effect of different compounds on cortical neurones previously exposed to glutamate**

| **Condition** | **Mean ± s.e.m (%)** |
|---|---|
| Control | 100 ± 4 |
| Glutamate [100 µM] | 66 ± 2 |
| Glutamate + 17β-oestradiol [3 nM] | 69 ± 2 |
| Glutamate + 17β-oestradiol [30 nM] | 75 ± 5 |
| Control | 100 ± 1 |
| Glutamate [100 µM] | 67 ± 3 |
| Glutamate + Sarsasapogenin [3 nM] | 101 ± 3 |
| Glutamate + Sarsasapogenin [30 nM] | 112 ± 1 |
| Glutamate + Smilagenin [3 nM] | 109 ± 6 |
| Glutamate + Smilagenin [30 nM] | 104 ± 1 |
| Control | 100 ± 8 |
| Glutamate [100 µM] | 40 ± 1 |
| Glutamate + Diosgenin [30 nM, negative control] | 49 ± 6 |
| Control | 100 ± 5 |
| Glutamate [100 µM] | 64 ± 4 |
| Glutamate + 16,22-epoxycoprostan-3β-ol [3 nM] | 114 ± 7 |
| Glutamate + 16,22-epoxycoprostan-3β-ol [30 nM] | 134 ± 5 |
| Glutamate + Smilagenone [3 nM] | 119 ± 7 |
| Glutamate + Smilagenone [30 nM] | 119 ± 4 |
| Control | 100 ± 4 |
| Glutamate [100 µM] | 58 ± 3 |
| Glutamate + Smilagenin glycinate | 117 ± 4 |
| hydrochloride [3 nM] | |
| Glutamate + Smilagenin glycinate | 141 ± 6 |
| hydrochloride [30 nM] | |
| Glutamate + Coprosterol [3 nM] | 126 ± 5 |
| Glutamate + Coprosterol [30 nM] | 116 ± 4 |

In conclusion, in rat primary cortical neurones sarsasapogenin, smilagenin, 16,22-epoxycoprostan-3β-ol, smilagenone, smilagenin glycinate hydrochloride and coprosterol reversed the neurodegeneration induced by glutamate, suggesting a therapeutic potential in neurodegenerative disorders.

### EXAMPLE 4

### Anti-apoptotic effect of smilagenin in neurones

The objective of this study was to examine the anti-apoptotic effect of smilagenin on the caspase-3 activity, a marker of apoptosis, in rat primary cortical cultures exposed to glutamate

### Primary cultures of cortical neurones

Rat cortical neurones were cultured for 6 days. At day 6, glutamate (100 microM, 10 min) was added. Then the cultures were washed and medium was replaced with fresh medium containing smilagenin or vehicle control (DMSO, 0.25%) for 6 h. After 6 h treatment, apoptosis was evaluated by measuring caspase 3, activity. Caspase 3 activity was detected by the cleavage of p-nitroaniline from a colorimetric caspase-3 substrate, acetyl-Asp-Glu-Val-Asp p-nitroanilide. p-Nitroalanine has a high absorbance at 405 nM. Relative caspase-3 activity was measured as optical density. In addition the relative activity of caspase-3 was standardised to protein concentration of the sample, which was also measured as an optical density (Du et al, J Neurochem., 69, 1382-1388, 1997; Sawada et al, Faseb J., 14, 1202-1214,2000).

Smilagenin reverses the increase in.glutamate induced caspase 3 activity in rat primary cortical neurones, demonstrating the anti-apoptotic effect of smilagenin (Table 4)

**Table 4 - Effect of smilagenin on glutamate induced caspase 3 activity in cortical neurones**

| **Conditions** | **Caspase activity (% of control)** |
|---|---|
| Control | 100 |
| + Glutamate | 131 |
| + Glutamate + smilagenin (300 nM) | 105 |

### EXAMPLE 5

Neurodegenerative disorders are characterised by a progressive loss of neurones and degradation of neuronal processes (neurites). Agents that induce neurite outgrowth may promote the formation of new connections between neurones and ameliorate the symptoms of neurodegenerative conditions (Katzman et al, Faseb J., 5, 278-286, 1991).

Exposure to 17β-oestradiol (0.3, 3, 30 pM) significantly increased the length of existing neurites in rat primary cortical neurones (Table 5). Exposure to 17β-oestradiol (3, 30 pM) significantly increased the percentage of neurones displaying neurites in rat primary cortical neurones (Table 6). Exposure to smilagenin and sarsasapogenin (0.3, 3, 30 pM) significantly increased the length of existing neurites and the percentage of neurones displaying neurites in rat primary cortical neurones (Tables 5 and 6).

In conclusion, smilagenin and sarsasapogenin have neurotrophic effects *in vitro.*

**Table 5 - Effect of 17β-oestradiol, smilagenin and sarsasapogenin on neurite length measured using optical micrometry**

| Conditions | Mean ± s.e.m (%) |
|---|---|
| Control | 100 ± 4 |
| 17β-oestradiol (0.3 pM) | 154 ± 5 |
| 17β-oestradiol (3 pM) | 163 ± 4 |
| 17β-oestradiol (30 pM) | 183 ± 5 |
| Smilagenin (0.3 pM) | 159 ± 5 |
| Smilagenin (3 pM) | 190 ± 8 |
| Smilagenin (30 pM) | 204 ± 6 |
| Sarsasapogenin (0.3 pM) | 177 ± 5 |
| Sarsasapogenin (3p M) | 197 ± 5 |
| Sarsasapogenin (30 pM) | 211 ± 6 |

**Table 6 - Effect of 17β-oestradiol, smilagenin and sarsasapogenin on the number of neurones displaying neurites**

| Conditions | Mean ± s.e.m (%) |
|---|---|
| Control | 47 ± 2 |
| 17β-oestradiol (0.3 pM) | 48 ± 2 |
| 17β-oestradiol (3 pM) | 60 ± 2 |
| 17β-oestradiol (30 pM) | 59 ± 2 |
| Smilagenin (0.3 pM) | 63 ± 2 |
| Smilagenin (3 pM) | 63 ± 2 |
| Smilagenin (30 pM) | 66 ± 2 |
| Sarsasapogenin (0.3 pM) | 55 ± 2 |
| Sarsasapogenin (3 pM) | 61 ± 1 |
| Sarsasapogenin (30 pM) | 62 ± 2 |

### EXAMPLE 6

Smilagenin and sarsasapogenin prevent the neurodegeneration caused by exposure to the neurotoxin, 1-methyl-4-phenylpyridinium (MPP⁺) in rat mesencephalic dopaminergic neurones; a model of Parkinson's disease *in vitro.*

Damage caused by the neurotoxin, MPP⁺, a metabolite of 1-methyl-4-phenyl-1,2,3,6-tetrahydropyridine (MPTP) mimics the degeneration of nigrostriatal dopaminergic neurones observed in neurodegenerative disorders such as Parkinson's disease (Mytinlineou et al, Science, 225, 529-531, 1984). The most prominent biochemical changes induced by this toxin include decreased levels of dopamine and its metabolites in the substantia nigra pars compacta and in the caudate nucleus (Burns et al, Proc Natl Acad Sci U.S.A., 80, 4546-4550, 1983) and a reduction in dopamine uptake in nigrostriatal synaptosomal preparations (Heikkila et al, J Neurochem., 44, 310-313, 1985).

Pre-treatment of dopaminergic neurones with smilagenin and sarsasapogenin significantly reduced the neuronal death following exposure of the dopaminergic specific neurotoxin MPP⁻ (2 µM⁻) when compared with MPP⁺ alone. Glial cell line-derived neurotrophic factor (GDNF) and brain-derived neurotrophic factor (BDNF), molecules that are involved in neuronal growth, were used as positive controls. Pre-treatment with smilagenin and sarsasapogenin produced a significant increase in the neuronal survival compared to neurones exposed to MPP⁺ alone, suggesting a significant neuroprotective effect (Table 7).

**Table 7 - Effect of pre-treatment with BDNF and GDNF, smilagenin and sarsasapogenin on dopaminergic neurones after MPP⁺ (2 µM) exposure**

| Condition | Mean ± s.e.m (%) |
|---|---|
| Control | 100 ± 3 |
| + MPP⁺ (2 µM) | 55 ± 3 |
| + MPP⁺ (2 µM)+ BDNF (1.85 nM) & GDNF (0.17 nM) | 122 ± 7 |
| + MPP⁺ (2 µM) + smilagenin (30 nM) | 98 ± 4 |
| + MPP⁺ (2 µM) + sarsasapogenin (30 nM) | 89 ± 3 |

In this model of Parkinson's disease *in vitro,* pre-treatment with smilagenin and sarsasapogenin significantly prevented the neuronal degeneration following exposure to the dopaminergic specific neurotoxin, MPP⁺, demonstrating a neuroprotective effect.

### EXAMPLE 7

Smilagenin and sarsasapogenin also reverse the neurodegeneration caused by exposure to the neurotoxin 1-methyl-4-phenylpyridinum (MPP⁺) in rat mesencephalic dopaminergic neurones; a model of Parkinson's disease *in vitro*.

Treatment of dopaminergic neurones with smilagenin and sarsasapogenin significantly reduced the neuronal death following exposure to the dopaminergic specific neurotoxin MPP⁺ (2 µM) when compared with MPP⁺ alone. Glial cell line-derived neurotrophic factor (GDNF) and brain-derived neurotrophic factor (BDNF), molecules that are involved in neuronal growth, and 17β-oestradiol were used as positive controls. Treatment with smilagenin and sarsasapogenin produced a significant increase in the neuronal survival compared to neurones exposed to MPP⁺ alone (Table 8).

**Table 8- Effect of treatment with BDNF and GDNF, smilagenin, sarsasapogenin and 17β-oestradiol on dopaminergic neurones after MPP⁺ (2 µM) exposure**

| Condition | Mean ± s.e.m (%) |
|---|---|
| Control | 100 ± 6 |
| + MPP⁺ (2 µM) | 76 ± 4 |
| + MPP⁺ (2 µM)+ BDNF (1.85 nM) & GDNF (0.17 nM) | 98 ± 5 |
| + MPP⁺ (2 µM) + smilagenin (0.03 nM) | 111 ± 6 |
| + MPP⁺ (2 µM) + sarsasapogenin (0.03 nM) | 112 ± 6 |
| + MPP⁺ (2 µM) + 17β-oestradiol (0.03 nM) | 106 ± 5 |

Exposure to MPP⁺ caused not only a significant decrease in dopaminergic number but also in the percentage of neurites. This study shows that smilagenin and sarsasapogenin significantly increased the number of neurites of the neurones *in vitro* Table 9. These results demonstrate that the compounds reverse motor neurodegeration.

**Table 9 - Effect of smilagenin and sarsasapogenin on percentage of neurites in dopaminergic neurones after MPP⁺ (2 µM) exposure**

| Condition | Mean ± s.e.m (%) |
|---|---|
| Control | 41 ± 4 |
| + MPP⁺ (2 µM) | 27 ± 5 |
| + MPP⁺ (2 µM) + smilagenin (0.03 nM) | 41 ± 4 |
| + MPP⁺ (2 µM) + sarsasapogenin (0.03 nM) | 43 ± 4 |

### EXAMPLE 8

### Neuroprotective effect of sarsasapogenin and smilagenin in spinal motor neurones

The objective of this study was to examine the effects of sarsasapogenin and smilagenin on the survival of rat primary spinal motor neurones exposed to glutamate, which is known to induce neurodegeneration in this model of motor neurodegeneration, 17β-oestradiol and BDNF were used as positive controls.

### Primary cultures of spinal motor neurones

Rat motor neurones were prepared according to the method described by (Martinou et al, Neuron, 8, 737-744, 1992). On day 10, medium was removed and the cultures were exposed to glutamate (4 microM) for 10 min at 37°C in defined medium. After the glutamate exposure, cultures were washed with Dulbecco modified Eagle medium at 37°C then placed in fresh culture medium containing test compounds. After 48 h, the extent of spinal motor neurone degeneration was determined by measuring the amount of lactate dehydrogenase (LDH) released into the culture medium as above.

### Results

Following exposure of glutamate, there was a significant degeneration of rat primary spinal motor neurones, 48 h post-treatment, demonstrated by an increase in lactate dehydrogenase release into the culture medium.

In primary rat primary spinal motor neurones treated with sarsasapogenin or smilagenin for 48 h, there was a significant reduction in the glutamate-induced neurodegeneration (Table 10).

**Table 10 - Effect of sarsasapogenin and smilagenin on glutamate-induced neurodegeneration in spinal motor neurones**

| Condition | Mean ± s.e.m (%) |
|---|---|
| Control + DMSO [0.25 %] | 100 ± 1 |
| Glutamate [4 microM] + DMSO [0.25 %] | 94 ± 1 |
| Glutamate + BDNF [3 nM] | 148 ± 8 |
| Glutamate + 17β-oestradiol [0.03 nM] | 102 ± 2 |
| Glutamate + 17β-oestradiol [3 nM] | 110 ± 1 |
| Glutamate + 17β-oestradiol [300 nM] | 116 ± 6 |
| Glutamate + sarsasapogenin [0.03 nM] | 123 ± 2 |
| Glutamate + sarsasapogenin [3 nM] | 137 ± 1 |
| Glutamate + sarsasapogenin [300 nM] | 136 ± 6 |
| Glutamate + smilagenin [0.03 nM] | 128 ± 4 |
| Glutamate + smilagenin [3 nM] | 154 ± 1 |
| Glutamate + smilagenin [300 nM] | 144 ± 4 |

Sarsasapogenin and smilagenin reversed the glutamate-induced neurodegeneration in rat spinal motor neurones in this *in vitro* model of motor neurodegeneration

### EXAMPLE 9

In the second half of life (in humans from the age of 40 onwards) the density of neurones in the brain decreases (Selkoe, D J, Sci. Am. 267, 134-142, 1992). The alterations in cortical function may be due to a reduction in the number of neurones, their interconnections, a decrease in neurotrophins such as brain derived neurotrophic factor (BDNF; Bothwell, M, Functional interactions of neurotrophins and neurotrophin receptors, Annu. Rev. Neurosci., 18, 223-253, 1995), a decrease in acetylcholine receptor density (muscarinic and nicotinic) and/or a decrease in their coupling function in cortical areas (Rinne et al, Brain Res., 336, 19-25, 1985; Selkoe, D J, Sci. Am. 267, 134-142, 1992). Furthermore during ageing, muscarinic acetylcholine receptor binding is significantly reduced in the hippocampus (Narang, N, Mech. Ageing Dev., 78, 221-239, 1995) and striatum of older rats (Biegon et al, Neurobiol. Aging., 10, 305-310,1989) and humans (Rinne et al, Brain Res., 336, 19-25, 1985). In addition, in Alzheimer's disease, the decline in cholinergic activity is associated with amyloid β plaque deposition (von der Kammer et al, Biochem. Soc. Symp. 131-140, 2001). Other neurodegenerative disorders, such as Parkinson's disease, show a characteristic decline in dopaminergic activity (Drukarch et al, Expert Opin. Investig. Drugs, 10, 1855-1868, 2001).

Oral administration of sarsasapogenin, episarsasapogenin cathylate or smilagenin to aged rats (Sprague-Dawley rats 20 month old), for a two or three month period reverses the impairment in learning and memory ability, the decline in muscarinic acetylcholine and dopamine receptors and the decline in the neurotrophin BDNF, alterations that are characteristic of the ageing process.

Aged Sprague-Dawley rats aged were divided into different groups, one control and groups treated for 2-3 months with either sarsasapogenin, episarsasapogenin cathylate or smilagenin (18 mg kg⁻¹ day⁻¹, n = 10). A control group (n=14) of untreated young rats was also included in the study. The daily dose of drug was mixed in a minimum amount of food and was administered every morning separately to each rat.

A Y-maze apparatus was used for the learning and memory test. On the floor of each arm of the Y-maze is an array of copper rods to which electric current is applied whenever needed, with adjustable voltage. Each arm is 45 cm long and has a 15 W lamp at the end, which is turned on when needed. After 3 months drug administration, each rat was trained for 7 consecutive days, as follows. For each training session, the rat was put into one arm of the Y-maze, after two minutes rest, an electrical current was applied to the copper rods and the lamp of the clockwise arm was illuminated to indicate the non-stimulation area. If the rat went into that arm, one correct response was recorded, otherwise, one wrong response was recorded. This stimulation-response test was repeated 20 times each day, with a pause of 5 sec between each two consecutive tests. The number of correct responses following the twenty tests on the seventh day was used to express learning ability, (the higher the number the better the learning ability). The rats were then left resting for 30 days and the procedure was repeated once more. The number of correct responses of the 20 tests after the 30 day rest period was used to represent the memory ability

Muscarinic acetylcholine receptor density in the brain was measured. Tissue was prepared as follows: brains were removed quickly after decapitation, frozen in dry ice, and transferred to a freezer. The brains were homogenised and the pellet was finally suspended in buffer.

The dual-site competitive ligand binding assay was used to measure muscarinic acetylcholine receptor density.

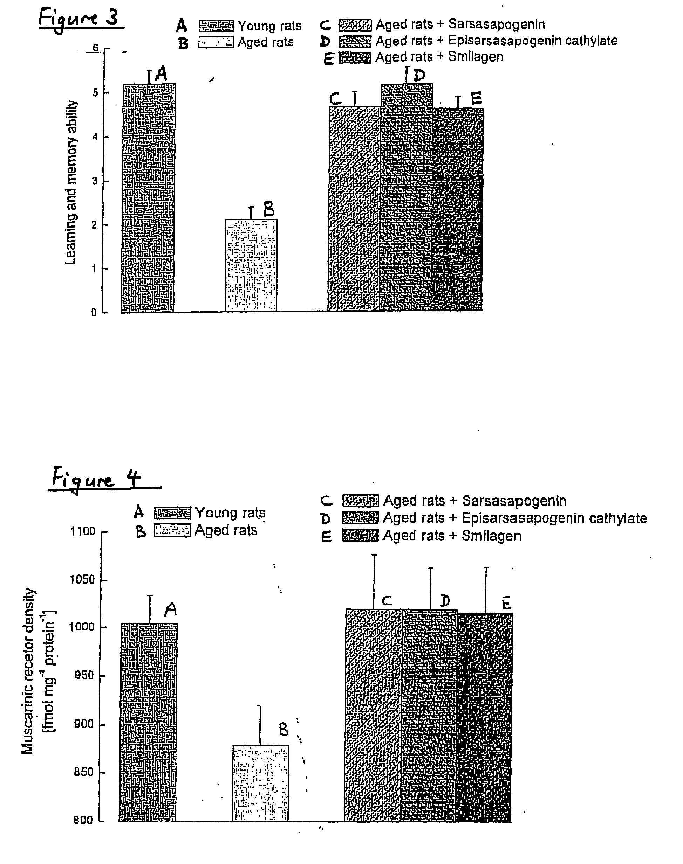

The results are shown in Figures 3 and 4 of the drawings. The Y-maze experiments revealed that both the learning ability and memory are impaired in aged rats. Sarsasapogenin, episarsasapogenin cathylate and smilagenin restored the learning and memory ability following administration in aged rats. Muscarinic acetylcholine receptor density was markedly reduced in aged rats. Sarsasapogenin, episarsasapogenin cathylate and smilagenin significantly restored the muscarinic acetylcholine receptor density.

Young rats showed a significantly higher dopamine (D) 1 and 2 receptor density (157.5 ± 33.2; 200.6 ± 50.9 fmol/mg protein, respectively) compared to aged rats (129.2 ± 36.8; 153.8 ± 40.5 fmol/mg protein, D₁ and D₂, respectively). By contrast, smilagenin and sarsasapogenin treatment in aged rats for 3 months restored the D₁ and D₂ receptor density (smilagenin 177 ± 10.9; 217 ± 45.7 fmol/mg protein; sarsasapogenin 172.0 ± 44.0; 206.4 ± 60.5 respectively).

Young rats showed significantly higher BDNF levels (1.647 ± 0.277 ng/g tissue) compared to aged rats (1.205 ± 0.219 ng/g tissue). By contrast, smilagenin and sarsasapogenin treatment in aged rats for 3 months, partially restored BDNF levels (1.342 ± 0.07; 1.410 ± 0.232 ng/g tissue, respectively).

Thus, the compounds reverse the neuroimpairment, the decline in BDNF levels, and the decline in the muscarinic acetylcholine and dopamine receptor density that occur in aged rats.

### EXAMPLE 10

### Alzheimer's disease model as a model of neurodegeneration

An *in vivo* model of Alzheimer's disease was used to model neurodegeneration. In this model the neurotoxic agents (amyloid β and ibotenic acid) are injected into the brain of the rat. This leads to neuronal loss, receptor loss and cognitive impairment. Previous studies showed that local injection of amyloid β in the nucleus vasalis of the rat brain caused cholinergic hypofunction and behavioural impairment up to two months post surgery (Giovannelli et al., 1995: Neuroscience, 66, 781-792.). In addition the co-injection of amyloid β with a small amount of ibotenic acid into the rat hippocampus synergistically produces neuronal loss with infiltration of glial cells not only adjacent but also far from the injected site (Morimoto et al., 1998: Neuroscience, 84, 479-487).

Our studies used the method of Morimoto (Morimoto et al., 1998: Neuroscience, 84, 479-487) with some modifications (unilateral instead of bilateral injection). Three months old, Sprague Dawley rats, were randomly divided into different groups. Injection of amyloid β₁-₄₀ and ibotenic acid (both from Sigma) was accomplished by means of a stereotaxic instrument (Stoelting Co.) and the coordinates were AP= -0.5mm (right to medial line), L= -2.8mm (backward from bregma), H= -7.0mm (ventral to dura). The dose for each rat was amyloid β₁₋₄₀(4 µg) and ibotenic acid (1 µg) in 1 µl of saline. The injection was completed in 20 min, and the needle was withdrawn 10 min later. Then the skin was sutured.

The 8 groups were:
Operated control injected with normal saline (control)
Model (control injected with amyloid β + ibotenic acid)
Model + Episarsasapogenin cathylate (18 mg/kg/day)*
Model + Sarsasapogenin cathylate (18 mg/kg/day)*
Model + Episarsasapogenin ethylsuccinate (18 mg/kg/day) (comparison)
Model + Episarsasapogenin (18 mg/kg/day)*
Model + Epismilagenin (18 mg/kg/day)*
Model + Diosgenin (i.e. negative control, 18 mg/kg/day)

* Compounds in accordance with the present invention

Episarsasapogenin cathylate, sarsasapogenin cathylate, episarsasapogenin ethylsuccinate (comparison compound), episarsasapogenin, epismilagenin and diosgenin (all at a dosage of 18 mg/kg/day) were administered to animals as stable suspensions in CMC-Na (0.5%) once daily through a gastric tube. The control and the model group were given the same volume of CMC-Na (0.5%) once daily. The drugs and vehicles were given for a period of two months, starting 20 days before operation.

Muscarinic acetylcholine receptor density was assessed. The brain samples were homogenised, centrifuged, and the pellet of centrifugation at 27000xg was re-homogenised and used for measurement. The concentration of ³H-QNB was chosen at the saturation range. After incubation and separation, the bound portion was measured by liquid scintillation counter.

Step-Through Test: learning and memory. The effect of test compounds on learning and memory was assessed using the step-through test. A 60 × 15 × 15 cm box, divided into 2 equally sized rooms, one dark room with copper rod base, which was electrically charged (40 V ac) when in use, while the other was a light room but not electrically charged. Between the two rooms there is an opening (hole) for the rat to go through. The experiment is carried out for each rat on two consecutive days. The first day is for training; when the rat is adapted in the box for the first 3 min, then put in the light room, with its back toward the hole, and the copper rods of the dark room are charged for 5 min. The second day is for testing, when the number of crosses in 5 min are recorded. Improvements in memory are signalled by a reduction in the number of crosses.

The muscarinic acetylcholine receptor density in the neurodegeneration model brains was significantly lower than control. Episarsasapogenin cathylate, sarsasapogenin cathylate, episarsasapogenin and epismilagenin produced a significant elevation in brain muscarinic acetylcholine receptor density, whereas diosgenin and episarsasapogenin ethylsuccinate did not significantly change the muscarinic acetylcholine receptor density. Thus the experiments indicate that the compounds of this invention act to normalise receptor number, i.e. they tend to restore receptor number to normal levels when given to animals in which the receptor level is depressed.

The number of wrong responses (error number) in 5 min was significantly higher in the neurodegeneration model group than the control group, indicating an impairment of memory (see Table 11). Epismilagenin, episarsasapogenin cathylate, episarsasapogenin and sarsasapogenin cathylate each significantly decreased the number of wrong responses, whereas diosgenin and episarsasapogenin ethylsuccinate were both ineffective in decreasing the number of wrong responses.

**Table 11**

| Group | M receptor density | Learning and memory Step through test |
|---|---|---|
| | (fmol/mg/protein) | Error No |
| Control (n=10) | 859±101 | 0.60±0.70 |
| Model (n=10) | 713±48 | 4.00±2.40 |
| + Episarsasapogenin cathylate (n= 10) | 877±89 * | 1.36±0.92* |
| + Sarsasapogenin cathylate (n=11) | 916±158 * | 1.36±1.03 * |
| + Episarsasapogenin ethylsuccinate (n=11) | 774±79 | 3.73±1.35 |
| + Episarsasapogenin (n=10) | 869±104* | 1.50±1.18* |
| + Epismilagenin (n=11) | 877±90* | 1.73±0.91* |
| + Diosgenin (negative control n=8) | 770±68 | 3.75±1.49 |

Statistical analysis using unpaired Student t test. * denotes P<0.05

### EXAMPLE 11

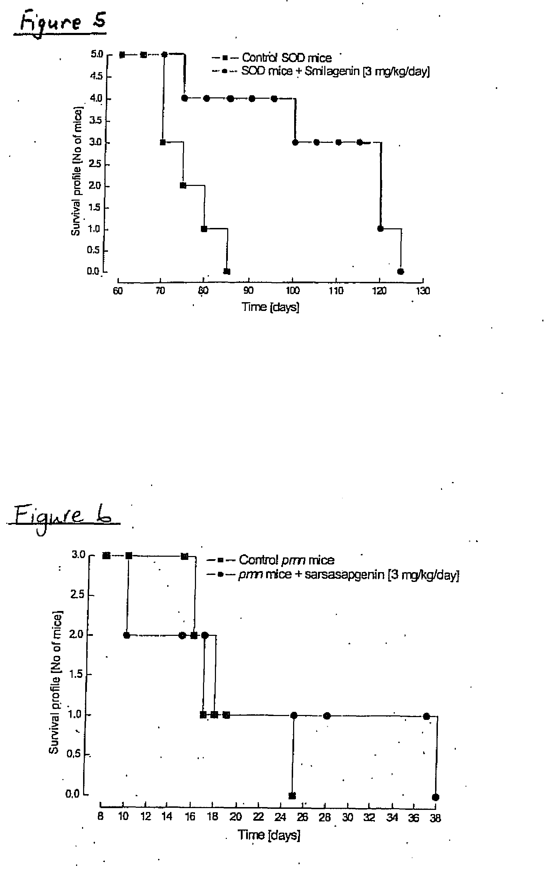

Amyotrophic lateral sclerosis (ALS) is a progressive fatal neurodegenerative disorder that causes motor neurone degeneration, skeletal atrophy, paralysis and death. The cause of this disease is heterogeneous: mutations in the Cu/Zn superoxide dismutase (SOD-1) gene is responsible for some forms of human ALS. Animal models of this disease include the SOD-1 transgenic mice over-expressing SOD-1 gene and the progressive motor neuropathy (pmn, a model of Charcot-Marie-Tooth) mice. Smilagenin and sarsasapogenin increase the lifespan and improve the behavioural deficits of the superoxide dismutase (SOD) mouse (Figure 5) and pmn mouse (Figure 6), two models of amyotrophic lateral sclerosis (ALS) and Charcot-Marie-Tooth disease.

The foregoing broadly describes the present invention without limitation. Variations and modifications as will be readily apparent to those of ordinary skill in this art are intended to be within the scope of this application and any subsequent patent(s).

## Claims

1. Use of one or more active agent selected from:
A. compounds of Formula I: 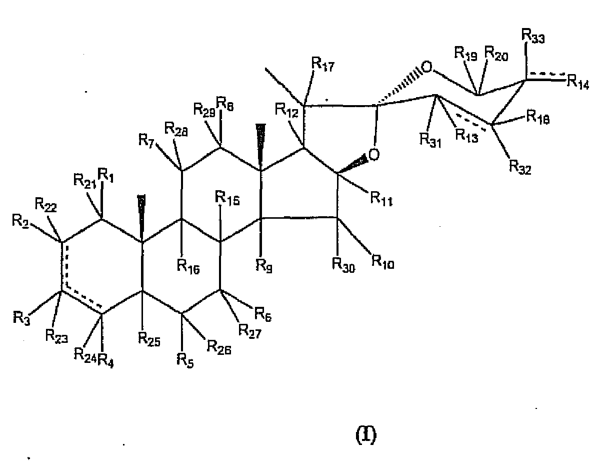 wherein in the general formula (I):
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂ are, independently of each other, either H, OH, =O, halo atom, (Me-S-), (Me-SO-), (Me-SO₂-), N₃-, NH₂-, MeSO₂NH-, alkyl or absent or OR where R = alkyl or acyl group;
- R₉, R₁₁, R₁₂, R₁₄, R₁₅, R₁₆, R₁₇, R₂₅, R₃₃ can be either a H, OH, halo atom, (Me-S-), (Me-SO-),(Me-SO₂-), N₃-, NH₂-, MeSO₂NH-, alkyl or absent or OR where R= alkyl or acyl group;
represents an optional double bond,
wherein in addition to the above
- either R₃₃ or R₁₄ = alkyl group;
B. compounds of Formula II: 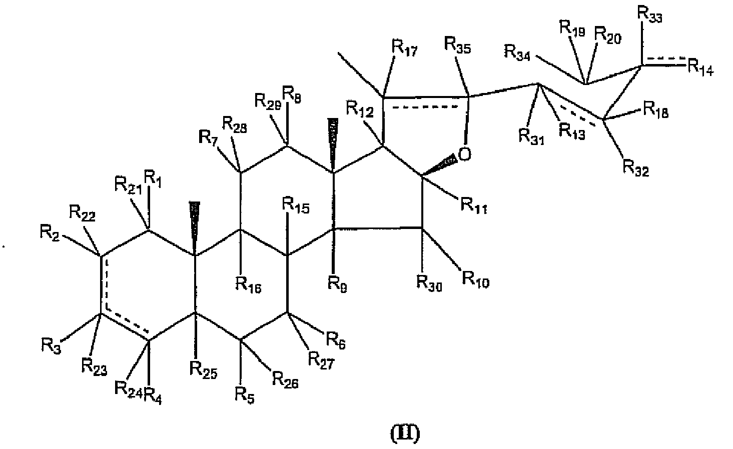 wherein in the general formula (II):
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂, R₃₄ are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
- R₉, R₁₁, R₁₂, R₁₄, R₁₅, R₁₆, R₁₇, R₂₅, R₃₃, R₃₅ can be either a H, OH, halo atom, (Me-S-), (Me-SO-), (Me-SO₂-), N₃-, NH₂-, MeSO₂NH-, alkyl, OR where R = alkyl or acyl group, or absent;
represents an optional double bond.
wherein in addition to the above
- either R₃₃ or R₁₄ = alkyl group;
C. compounds of Formula III: 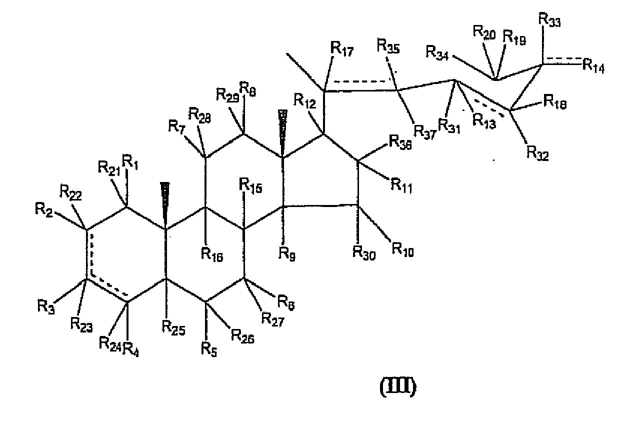 wherein in the general formula (III):
- R₁, R₂, R₃, R₄, R₅, R₆, R₇, R₈, R₁₀, R₁₃, R₁₄, R₁₈, R₁₉, R₂₀, R₂₁, R₂₂, R₂₃, R₂₄, R₂₆, R₂₇, R₂₈,
R₂₉, R₃₀, R₃₁, R₃₂, R₃₃, R₃₄, R₃₅, R₃₆, R₃₇ are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
- R₉, R₁₁, R₁₂, R₁₅, R₁₆, R₁₇, R₂₅ can be either H, OH, halo atom, (Me-S-), (Me-SO-), (Me-SO₂), N₃-, NH₂-, MeSO₂NH-, alkyl, OR where R = alkyl or acyl group, or absent;
..... represents an optional double bond,
wherein in addition to the above
- either R₃₃ or R₁₄ = alkyl group, and
the stereochemistry of R₂₅ is in the β orientation;
D. sapogenin derivatives bearing at least one X radical substituent,
wherein X is chosen from the group consisting of :
- halo atom,
- (Me-S-), (Me-SO-), (Me-SO₂-),
- N₃-, NH₂-, MeSO₂NH-, and
- alkyl; and
E. derivative forms of any of the above compounds, in which the carbon atom at the 3-position or, in the case of Formulae II and III, the 3-position carbon atom, the 26-position or each of the carbon atoms at the 3- and 26-positions, carries an O-sugar moiety wherein the sugar group is a mono-, di- or tri-saccharide;
all their stereoisomers and racemic mixtures, all their pharmaceutically acceptable prodrugs and salts, and all mixtures and combinations thereof
in the treatment or prevention of, or in the preparation of compositions for the treatment or prevention of, (i) non-cognitive neurodegeneration, (ii) non-cognitive neuromuscular degeneration, (iii) motor-sensory neurodegeneration, or (iv) receptor dysfunction or loss in the absence of cognitive, neural and neuromuscular impairment, in human and non-human animals suffering therefrom or susceptible thereto.

2. A use according to claim 1, wherein the active agent, or at least one of the active agents, is selected from:
a. Compounds of the above general formula I, wherein:
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂ are, independently of each other, either H, OH, =O, halo atom, (Me-S-), (Me-SO-), (Me-SO₂-), N₃-, NH₂-, MeSO₂NH-, alkyl or absent or OR where R = alkyl or acyl group;
- R₉, R₁₁, R₁₂, R₁₄, R₁₅, R₁₆, R₁₇, R₂₅, R₃₃ can be either a H, OH, halo atom, (Me-S-), (Me-SO-), (Me-SO₂-), N₃-, NH₂-, MeSO₂NH-, alkyl or absent or OR where R= alkyl or acyl group;
..... represents an optional double bond,
wherein in addition to the above
- either R₃₃ or R₁₄ = alkyl, group,
and the stereochemistry of R₂₅ is in the β orientation;
b. Compounds of the above general formula I, wherein:
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂ are, independently of each other, either H, OH, =O, halo atom, (Me-S-), (Me-SO-), (Me-SO₂), N₃-, NH₂-, MeSO₂NH-, alkyl or absent or OR where R = alkyl or acyl group;
- R₉, R₁₂, R₁₅, R₁₆, R₁₇ = H,
- R₁₁, R₁₄, R₂₅, R₃₃ can be either a H, OH, halo atom, (Me-S-), (Me-SO-), (Me-SO₂-), N₃-, NH₂-, MeSO₂NH-, alkyl or absent or OR where R = alkyl or acyl group;
..... represents an optional double bond
wherein in addition to the above
- either R₃₃ or R₁₄ = alkyl group, and the stereochemistry of R₂₅ is in the β orientation;
c. Compounds of the above general formula I, wherein:
- R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
R₂₁= R₂₂= R₂₃= R₂₄= R₂₅= R₂₆= R₂₇= R₂₈=R₂₉= R₃₀= R₃₁= R₃₂= R₃₃ = H,
- either R₃₃ or R₁₄ = CH₃
..... represents a single bond,
- the methyl group at C25 may be either in the R or S configuration
- the stereochemistry of R₂₅ is in the β orientation and
wherein in addition to the above
at least one of R₃ or R₂₃ is a X radical, the possible remaining substituent being H, OH, =O, and OR where R = alkyl or acyl group or absent, and X is chosen from the group consisting of :
- halo atom,
- (Me-S-), (Me-SO-), (Me-SO₂-), and
- N₃-, NH₂-, MeSO₂NH-
- alkyl;
d. Compounds of the above general formula I, wherein:
- R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
R₂₁= R₂₂= R₂₃= R₂₄= R₂₅= R₂₆= R₂₇= R₂₈=R₂₉= R₃₀= R₃₁= R₃₂ = H,
- R₁₄= R₃₃ = CH₃,
..... represents a single bond,
- the stereochemistry of R₂₅ is in the β orientation and
wherein in addition to the above
at least one of R₃ or R₂₃ is a X radical, the possible remaining substituent being H, OH, =0, and OR where R = alkyl or acyl group or absent,
and X is chosen from the group consisting of :
- halo atom,
- (Me-S-), (Me-SO-), (Me-SO₂-), and
- N₃-, NH₂-, MeSO₂NH-
- alkyl;
e. Compounds of the above general formula II, wherein
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂, R₃₄ are, independently of each other, either H, OH, =O, halo atom, (Me-S-), (Me-SO-), (Me-SO₂), N₃-, NH₂-, MeSO₂NH-, alkyl, OR where R = alkyl or acyl group, or absent;
- R₉, R₁₁, R₁₂, R₁₄, R₁₅, R₁₆, R₁₇, R₂₅, R₃₃, R₃₅ can be either a H, OH, halo atom, (Me-S-), (Me-SO-), (Me-SO₂-), N₃-, NH₂-, MeSO₂NH-, alkyl, OR where R = alkyl or acyl group, or absent;
..... represents an optional double bond,
wherein in addition to the above
- either R₃₃ or R₁₄ = alkyl group, and the stereochemistry of R₂₅ is in the β orientation;
f. Compounds of the above general formula II or carbohydrate derivatives thereof, wherein:
- R₁, R₂, R₃, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉,
R₃₀, R₃₁, R₃₂ are, independently of each other, either H, OH, =O, halo atom, (Me-S-), (Me-SO-), (Me-SO₂), N₃-, NH₂-, MeSO₂NH-, alkyl, OR where R = alkyl or acyl group, or absent;
- R₉, R₁₂, R₁₅, R₁₆, R₁₇ = H,
- R₃₄= either H, OH, =O, and OR where R = alkyl, acyl or carbohydrate and
- R₁₁, R₁₄, R₂₅, R₃₃, R₃₅ can be either H, OH, halo atom, (Me-S-), (Me-SO-), (Me-SO₂-), N₃-, NH₂-, MeSO₂NH-, alkyl, OR where R = alkyl or acyl group, or absent;
..... represents an optional double bond,
wherein in addition to the above
- either R₃₃ or R₁₄ = alkyl group, and the stereochemistry of R₂₅ is in the β orientation;
g. Compounds of the above general formula II or carbohydrate derivatives thereof, wherein:
- R₁= R₂= R₄= R₅= R₆= R₇= R₈= R₁₀= R₁₁= R₉= R₁₂= R₁₃= R₁₅ = R₁₆ = R₁₇ = R₁₈ = R₁₉ = R₂₀ =
R₂₁= R₂₂= R₂₃= R₂₄= R₂₅= R₂₆= R₂₇= R₂₈=R₂₉= R₃₀= R₃₁= R₃₂= R₃₃ = H,
- R₁₄ = CH₃,
- R₃₄= -OH or -OR where R = alkyl, acyl or carbohydrate and
R₃₅ = H or is absent
..... represents an optional double bond, and
- the methyl group at C25 may be either in the R or S configuration and
and the stereochemistry of R₂₅ is in the β orientation
wherein in addition to the above
at least one of R₃ or R₂₃ is a X radical, the possible remaining substituent being H, OH, =O, and OR where R = alkyl or acyl group or absent,
and X is chosen from the group consisting of:
- halo atom,
- (Me-S-), (Me-SO-), (Me-SO₂-), and
- N₃-, NH₂-, MeSO₂NH-
- alkyl;
h. Compounds of the above general formula II or carbohydrate derivatives thereof, wherein:
- R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
R₂₁= R₂₂= R₂₃= R₂₄= R₂₅= R₂₆= R₂₇= R₂₈=R₂₉= R₃₀= R₃₁= R₃₂= H,
- R₁₄ = R₃₃ = CH₃,
- R₃₄= -OH or -OR where R = alkyl, acyl or carbohydrate and
R₃₅ = H or is absent
..... represents an optional double bond, and
the stereochemistry of R₂₅ is in the β orientation and
wherein in addition to the above
at least one of R₃ or R₂₃ is a X radical, the possible remaining substituent being H, OH, =O, and OR where R = alkyl or acyl group or absent,
and X is chosen from the group consisting of :
- halo atom,
- (Me-S-), (Me-SO-), (Me-SO₂-), and
- N₃-, NH₂-, MeSO₂NH-
- alkyl;
i. Compounds of the above general formula III, wherein:
- R₁, R₂, R₃, R₄, R₅, R₆, R₇, R₈, R₁₀, R₁₃, R₁₄, R₁₈, R₁₉, R₂₀, R₂₁, R₂₂, R₂₃, R₂₄, R₂₆, R₂₇, R₂₈,
R₂₉, R₃₀, R₃₁, R₃₂, R₃₃, R₃₄, R₃₅, R₃₆, R₃₇ are, independently of each other, either H, OH, =O, halo atom, (Me-S-), (Me-SO-), (Me-SO₂-), N₃-, NH₂-, MeSO₂NH-, alkyl, OR where R = alkyl or acyl group, or absent;
- R₉, R₁₁, R₁₂, R₁₅, R₁₆, R₁₇, R₂₅ can be either H, OH, halo atom, (Me-S-), (Me-SO-), (Me-SO₂), N₃-, NH₂-, MeSO₂NH-, alkyl, OR where R = alkyl or acyl group, or absent;
..... represents an optional double bond,
wherein in addition to the above
- either R₃₃ or R₁₄ = alkyl group, and the stereochemistry of R₂₅ is in the β orientation;
j. Compounds of the above general formula III or carbohydrate derivatives thereof, wherein:
- R₁, R₂, R₃, R₄, R₅, R₅, R₇, R₈, R₁₀, R₁₃, R₁₄, R₁₈, R₁₉, R₂₀, R₂₁, R₂₂, R₂₃, R₂₄, R₂₆, R₂₇, R₂₈,
R₂₉, R₃₀, R₃₁, R_{32,} R₃₃, R₃₅, R₃₆, R₃₇ are, independently of each other, either H, OR, =O, halo atom, (Me-S-), (Me-SO-), (Me-SO₂-), N₃-, NH₂-, MeSO₂NH-, alkyl, OR where R = alkyl or acyl group, or absent;
- R₉, R₁₂, R₁₅, R₁₆, R₁₇ = H,
- R₃₄ = H, OH, =O, halo atom, (Me-S-), (Me-SO-), (Me-SO₂-), N₃-, NH₂-, MeSO₂NH-, alkyl, OR where R = alkyl, acyl or carbohydrate, or absent;
- R₁₁, R₂₅, can be either H, OH, halo atom, (Me-S-), (Me-SO-), (Me-SO₂-), N₃-, NH₂-, MeSO₂NH-, alkyl, OR where R = alkyl or acyl group, or absent;
..... represents an optional double bond,
wherein in addition to the above
- either R₃₃ or R₁₄ = alkyl group, and the stereochemistry of R₂₅ is in the β orientation;
k. Compounds of the above general formula III,wherein:
- R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ = R₂₀ =
R₂₁= R₂₂= R₂₃= R₂₄= R₂₅= R₂₆= R₂₇= R₂₈=R₂₉= R₃₀= R₃₁= R₃₂= R₃₃ = H,
- R₁₄ = CH₃,
- R₃₄= -OH or -OR where R = alkyl, acyl or carbohydrate and
R₃₅ = H or is absent
R₃₇ = H, -OH or =O
R₃₆= H or -OH
..... represents a single bond, and
- the methyl group at C25 may be either in the R or S configuration and
the stereochemistry of R₂₅ is in the β orientation
wherein in addition to the above
at least one of R₃ or R₂₃ is a X radical, the possible remaining substituent being H, OH, =O, and OR where R = alkyl or acyl group or absent,
and X is chosen from the group consisting of :
- halo atom,
- (Me-S-), (Me-SO-), (Me-SO₂-), and
- N₃-, NH₂-, MeSO₂NH-
- alkyl;
l. Compounds of the above general formula III or carbohydrate derivatives thereof, wherein:
- R₁= R₂= R₄= R₅= R₆= R₇= R₈= R₁₀=R₁₁= R₉= R₁₂= R₁₃= R₁₅ = R₁₆ = R₁₇ = R₁₈ = R₁₉ =R₂₀ =
R₂₁= R₂₂= R₂₃= R₂₄= R₂₅= R₂₆= R₂₇= R₂₈=R₂₉= R₃₀= R₃₁= R₃₂ = R₁₉ = R₂₀ = H,
- R₁₄= R₃₃ = CH₃,
- R₃₄= -OH or -OR where R = alkyl, acyl or carbohydrate and
R₃₅= H or is absent
R₃₇ = H, -OH or =O
R₃₆= H or -OH
..... represents a single bond, and
- the methyl group at C25 may be either in the R or S configuration and
the stereochemistry of R₂₅ is in the β orientation
wherein in addition to the above
at least one of R₃ or R₂₃ is a X radical, the possible remaining substituent being H, OH, =O, and OR where R = alkyl or acyl group or absent,
and X is chosen from the group consisting of :
- halo atom,
- (Me-S-), (Me-SO-), (Me-SO₂-), and
- N₃-, NH₂-, MeSO₂NH-
- alkyl;
m. Substituted sapogenins wherein at least one OH-group of the sapogenin is substituted with X, chosen from the group consisting of :
- halo atom,
- (Me-S-), (Me-SO-), (Me-SO₂-),
- N₃-, NH₂-, MeSO₂NH-, and
- alkyl;
n. Sapogenins defined above wherein in the definition of X the halo atom is a fluoro atom;
o. Substituted sapogenins selected from :
(3β-fluoro-5β, 20α,22α,25R-spirostane), (3,3-difluoro-5β, 20α,22α,25R-spirostane), (3α-methylsulphonylamino-5β,20α,22α,25R-spirostane), (3α-azido-5β, 20α,22α,25R-spirostane), (3α-amino-5β,20α,22α,25R-spirostane), and their stereoisomers and racemic mixtures, their pharmaceutically acceptable pro-drugs and salts;
p. Substituted sapogenins wherein the parent sapogenin which is then substituted with at least one X radical as defined above is selected from sarsasapogenin, episarsasapogenin, smilagenin, epismilagenin, and anzurogenin-D;
q. Compounds of the general formula Ia: 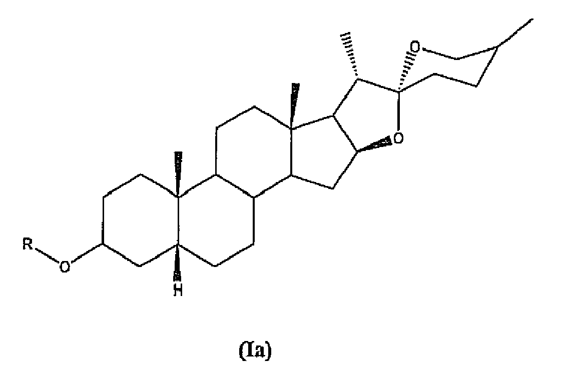 wherein the group R is selected from hydrogen; alkylcarbonyl; alkoxycarbonyl; alkylcarbamoyl; or arylcarbonyl; or sulpho (HO₃S); phosphono ((HO)₂P(O)-); or a mono-, di- or tri-saccharide; wherein any alkyl group is optionally substituted with aryl, amino, mono- or di-alkyl-amino, a carboxylic acid residue (-COOH), or any combination thereof; and
r. Derivative forms of the above compounds as defined as items a to q, in which the 3-position carbon atom or, in the case of Formulae II and III, the 3-position carbon atom, the 26-position carbon atom or each of the carbon atoms at the 3- and 26-positions, carries an O-sugar moiety wherein the sugar group is a mono-, di- or tri-saccharide, and acylated derivatives thereof.

3. A use according to claim 1 or claim 2, wherein the active agent, or at least one of the active agents, is selected from compounds of the general formula Ia.

4. A use according to any one of the preceding claims, wherein the active agent, or at least one of the active agents, is selected from:
sarsasapogenin
sarsasapogenin cathylate
sarsasapogenin acetate
sarsasapogenin succinate and pharmaceutically acceptable salts thereof
sarsasapogenin glycinate and pharmaceutically acceptable salts thereof
sarsasapogenin alaminate and pharmaceutically acceptable salts thereof
sarsasapogenin valinate and pharmaceutically acceptable salts thereof
sarsasapogenin phenylalaninate and pharmaceutically acceptable salts thereof
sarsasapogenin isoleucinate and pharmaceutically acceptable salts thereof
sarsasapogenin methioninate and pharmaceutically acceptable salts thereof
episarsasapogenin
episarsasapogenin cathylate
episarsasapogenin acetate
episarsasapogenin succinate and pharmaceutically acceptable salts thereof
episarsasapogenin glycinate and pharmaceutically acceptable salts thereof
episarsasapogenin alaninate and pharmaceutically acceptable salts thereof
episarsasapogenin valinate and pharmaceutically acceptable salts thereof
episarsasapogenin phenylalaninate and pharmaceutically acceptable salts thereof
episarsasapogenin isoleucinate and pharmaceutically acceptable salts thereof
episarsasapogenin methioninate and pharmaceutically acceptable salts thereof
smilagenin
smilagenin cathylate
smilagenin acetate
smilagenin succinate and pharmaceutically acceptable salts thereof
smilagenin glycinate and pharmaceutically acceptable salts thereof
smilagenin alaninate and pharmaceutically acceptable salts thereof
smilagenin valinate and pharmaceutically acceptable salts thereof
smilagenin phenylalaninate and pharmaceutically acceptable salts thereof
smilagenin isoleucinate and pharmaceutically acceptable salts thereof
smilagenin methioninate and pharmaceutically acceptable salts thereof
epismilagenin
epismilagenin cathylate
epismilagenin acetate
epismilagenin succinate and pharmaceutically acceptable salts thereof
epismilagenin glycinate and pharmaceutically acceptable salts thereof
epismilagenin alaninate and pharmaceutically acceptable salts thereof
epismilagenin valinate and pharmaceutically acceptable salts thereof
epismilagenin phenylalaninate and pharmaceutically acceptable salts thereof
epismilagenin isoleucinate and pharmaceutically acceptable salts thereof
epismilagenin methioninate and pharmaceutically acceptable salts thereof.
saponin derivatives of sarsasapogenin, episarsasapogenin, smilagenin and epismilagenin in which, in each case, the 3-position carbon atom carries an O-sugar moiety wherein the sugar group is selected from glucose, mannose, fructose, galactose, maltose, cellobiose, sucrose, rhamnose, xylose, arabinose, fucose, quinovose, apiose, lactose, galactose-glucose, glucose-arabinose, fucose-glucose, rhamnose-glucose, glucose-glucose-glucose, glucose-rhamnose, mannose-glucose, glucose-(rhamnose)-glucose, glucose-(rhamnose)-rhamnose, glucose-(glucose)-glucose, galactose-(rhamnose)-galactose and acylated derivatives thereof;
16,22-epoxycoprostan-3β-ol, smilagenone, coprosterol, and pharmaceutically acceptable pro-drugs and salts thereof.

5. A use according to any one of the preceding claims, wherein the active agent is present in a composition selected from pharmaceutical compositions, foodstuffs, food supplements and beverages.

6. A use according to any one of the preceding claims, wherein the active agent is present with one or more additional active agent.

7. A use according to claim 6, wherein the one or more additional active agent is selected from, but not limited, to cholinesterase inhibitors, dopamine agonists, COMT inhibitors, MAO-B inhibitors, anti-cholinergics, acetylcholine agonists, serotonin agonists, AMPA receptor agonists, GABA receptor agonists, NMDA receptor agonists, β-adrenceptor agonists, digoxin, dobutamine, anti-inflammatories, neurotrophic factors, statins, adenosine A2a receptor antagonists, aldose reductase inhibitors, immunomodulators, cannabinoid agonists, interferon β or tricyclic anti-depressants.

8. A use according to any one of the preceding claims, wherein the human or non-human animal is suffering from, or is susceptible to, any of: Parkinson's disease, postencephalitic Parkinsonism, depression, schizophrenia, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, motor neurone diseases including amyotrophic lateral sclerosis (ALS), multiple sclerosis, postural hypotension, traumatic neurodegeneration e.g. following stroke or following an accident (for example, traumatic head injury or spinal cord injury), Batten's disease, Cockayne syndrome, Down syndrome, corticobasal ganglionic degeneration, multiple system atrophy, cerebral atrophy, olivopontocerebellar atrophy, dentatorubral atrophy, pallidoluysian atrophy, spinobulbar atrophy, optic neuritis, sclerosing pan-encephalitis (SSPE), attention deficit disorder, post-viral encephalitis, post-poliomyelitis syndrome, Fahr's syndrome, Joubert syndrome, Guillain-Barre syndrome, lissencephaly, Moyamoya disease, neuronal migration disorders, autistic syndrome, polyglutamine disease, Niemann-Pick disease, progressive multifocal leukoencephalopathy, pseudotumor cerebri, Refsum disease, Zellweger syndrome, supranuclear palsy, Friedreich's ataxia, spinocerebellar ataxia type 2, Rhett syndrome, Shy-Drager syndrome, tuberous sclerosis, Pick's disease, chronic fatigue syndrome, neuropathies including hereditary neuropathy, diabetic neuropathy and mitotic neuropathy, prion-based neurodegeneration, including Creufefeldt-Jakob disease (CJD), variant CJD, new variant CJD, bovine spongiform encephalopathy (BSE), GSS, FFI, kuru and Alper's syndrome, Joseph's disease, acute disseminated encephalomyelitis, arachnoiditis, vascular lesions of the central nervous system, loss of extremity neuronal function, Charcot-Marie-Tooth disease, susceptibility to heart failure, asthma, and macular degeneration.
